# EUROPEAN PATENT APPLICATION

(11) **EP 4 700 061 A1**
(43) Date of publication of application: **25.02.2026**
(21) Application number: 24195939.4
(22) Date of filing: 22.08.2024
(51) Int. Cl.: C08G 59/50, C07C 213/02, C07C 217/08, C07C 213/04

(54) **PROCESS FOR THE SYNTHESIS OF FUNCTIONALIZED CLEAVABLE AMINES VIA ALKOXYLATION FOLLOWED BY ALCOHOL AMINATION**

(71) Applicant: BASF SE, 67056 Ludwigshafen am Rhein (DE)
(72) Inventor: SIEBERT, Max Julian, 67056 Ludwigshafen am Rhein (DE); ERNST, MArtin, 67056 Ludwigshafen am Rhein (DE); VANDERMEULEN, Guido, 67056 Ludwigshafen am Rhein (DE); TUERK, Holger, 67056 Ludwigshafen am Rhein (DE); ROTH, Torsten, 67056 Ludwigshafen am Rhein (DE); LANGLOTZ, Bjoern, 67056 Ludwigshafen am Rhein (DE)
(74) Representative: Altmann Stößel Dick Patentanwälte PartG mbB

(57) **Abstract**

A process for preparing polyamines via alkylene oxide addition to an amine followed by alcohol amination, comprising
a) reacting at least one amine of general formula (I) 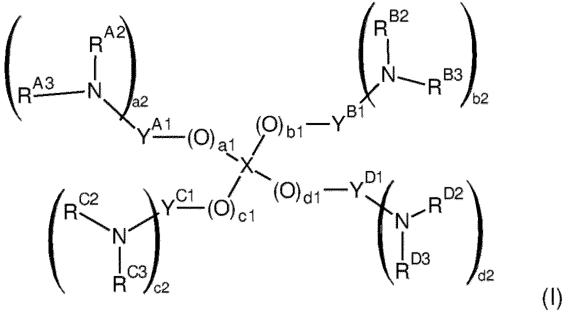 with at least one C2-C4-alkylene oxide, thereby obtaining at least one first intermediate (11), wherein the amine of general formula (I) comprises at least one, preferably terminal, group NH which is reacted with the C2-C4-alkylene oxide, thereby obtaining at least one first intermediate (11), wherein at least one, preferably terminal, group NH of the amine of general formula (I) is converted to at least one group N-CH₂-CHR-OH or N-CHR-CH₂-OH with R being H or methyl or ethyl, preferably N-CH₂-CH₂-OH;
b) aminating the at least one first intermediate (I1) with at least one of ammonia, primary and secondary amines HNR'R" with R' being H, methyl, ethyl, propyl or isopropyl and R" being H, methyl, ethyl, propyl or isopropyl, or mixtures thereof, to form at least one polyamine compound (II).

## Description

The present invention relates to a process for preparing functionalized cleavable amines via alkoxylation of amines followed by alcohol amination. Further, the present invention relates to an intermediate which is obtainable or obtained by said process and a polyamine compound which is obtainable or obtained by said process. Yet further, the present invention relates to the use of said polyamine compound in or as co-reactant, hardener or curative for epoxy resins and an epoxy resin composition containing such polyamine compound. Still further, the present invention relates to the use of said polyamine compound in specific applications.

The recycling of products or materials containing epoxy resins is still unsatisfactory since no commercial solutions have been realized yet and the market strives for enabling technologies. So far, the recycling of e.g. wind turbine rotor blades is still unsatisfactory and unsatisfying with regard to the epoxy system component.

Furthermore, for polyamines and products derived therefrom there are no solutions yet on how to improve the biodegradability of the polymers currently used.

EP 1391448 A1 describes the synthesis of functionalized amines with aliphatic backbone and no cleavable functionality via addition of acrylonitrile to diamines, followed by hydrogenation.

EP 1955997 A1 describes the reaction of ethylene diamine with acrylonitrile in the presence of a polar protic solvent followed by hydrogenation.

There is a general need for cleavable functionalities within amines for various applications. For example, customers are seeking improved recyclability of epoxy systems in wind turbine rotor blades or better biodegradable polymer additives based on polyamines.

Cleavable (poly)amines potentially represent a technology for solving this problem for the first time. This invention discloses a process for producing cleavable amines and describes various compounds that can be produced using this process. According to this process, the functionalized cleavable amine is synthesized in a two-step process. Starting from a cleavable amine and an alkylene oxide like ethylene oxide, an addition reaction is used to synthesize a product bearing at least one hydroxyl group as the desired intermediate for amination. This intermediate is then subjected to catalytic amination to yield the functionalized cleavable amine.

Therefore, the present invention relates to a process for preparing polyamines via alkylene oxide addition to an amine followed by alcohol amination, comprising
a) reacting at least one amine of general formula (I) 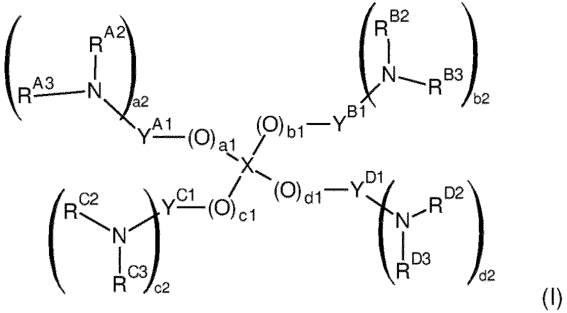 with at least one C2-C4-alkylene oxide, thereby obtaining at least one first intermediate (!1), wherein the amine of general formula (I) comprises at least one, preferably terminal, group NH which is reacted with the C2-C4-alkylene oxide, thereby obtaining at least one first intermediate (11), wherein at least one terminal group NH of the amine of general formula (I) is converted to at least one group N-CH₂-CHR-OH or N-CHR-CH₂-OH with R being H or methyl or ethyl, preferably N-CH₂-CH₂-OH;
b) aminating the at least one first intermediate (11) with at least one of ammonia, primary and secondary amines HNR'R" with R' being H, methyl, ethyl, propyl or isopropyl and R" being H, methyl, ethyl, propyl or isopropyl, or mixtures thereof, to form at least one polyamine compound (II), comprising converting the at least one group N-CH₂-CHR-OH or N-CHR-CH₂-OH, preferably the at least one group N-CH₂-CH₂-OH to the at least one group N-CH₂-CHR-NR'R" or N-CHR-CH₂-NR'R", preferably to the at least one group N-CH₂-CH₂-NH₂; and
   if at least one group N(-CH₂-CHR-OH)₂, N(-CHR-CH₂-OH)₂, or N(-CHR-CH₂-OH)(-CH₂-CHR-OH), preferably at least one group N(-CH₂-CH₂-OH)₂, is present in the at least one first intermediate (11) and at least one of ammonia or primary amines H₂NR' with R' being H, methyl, ethyl, propyl or isopropyl, or mixtures thereof, are employed in step b),
   then step b) further comprises the step of converting the at least one group N(-CH₂-CHR-OH)₂, N(-CHR-CH₂-OH)₂, or N(-CHR-CH₂-OH)(-CH₂-CHR-OH), preferably at least one group N(-CH₂-CH₂-OH)₂, in the at least one first intermediate (!1) with ammonia or primary amine H₂NR' with R' being H, methyl, ethyl, propyl or isopropyl, or mixtures thereof, to form at least one, optionally substituted, piperazine group -N(-CH₂-CHR-)₂NR', -N(-CHR-CH₂-)₂NR' or -N(-CH₂-CHR-)(-CHR-CH₂-)NR', preferably to form at least one piperazine group -N(-CH₂-CH₂-)₂NH as shown below; 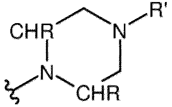 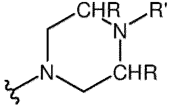 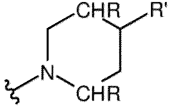 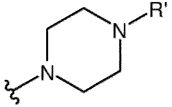
   wherein X is C or Si or HC-CH;
   wherein a1, a2, b1, b2, c1, c2, d1, d2 are independently of each other 0 or 1, with a1 + b1 + c1 + d1 ≥ 2 and a2 + b2 + c2 + d2 ≥ 2;
   wherein, if a1 = a2 = 0, Y^{A1} is H or optionally substituted C1-C12 alkyl;
   wherein, if a1 = 1 and a2 = 0, Y^{A1} is optionally substituted C1-C12 alkyl;
   wherein, if a1 = a2 = 1, or if a1 = 0 and a2 = 1, Y^{A1} is optionally substituted C1-C12 alkylene;
   wherein if b1 = b2 = 0, Y^{B1} is H or optionally substituted C1-C12 alkyl;
   wherein if b1 = 1 and b2 = 0, Y^{B1} is optionally substituted C1-C12 alkyl;
   wherein if b1 = b2 = 1, or if b1 = 0 and b2 = 1, Y^{B1} is optionally substituted C1-C12 alkylene;
   wherein if c1 = c2 = 0, Y^{C1} is H or optionally substituted C1-C12 alkyl;
   wherein if c1 = 1 and c2 = 0, Y^{C1} is optionally substituted C1-C12 alkyl;
   wherein if c1 = c2 = 1, or if c1 = 0 and c2 = 1, Y^{C1} is optionally substituted C1-C12 alkylene;
   wherein if d1 = d2 = 0, Y^{D1} is H or optionally substituted C1-C12 alkyl;
   wherein if d1 = 1 and d2 = 0, Y^{D1} is optionally substituted C1-C12 alkyl;
   wherein if d1 = d2 = 1, or if d1 = 0 and d2 = 1, Y^{D1} is optionally substituted C1-C12 alkylene;
   wherein R^{A2}, R^{A3}, R^{B2}, R^{B3}, R^{C2}, R^{C3}, R^{D2}, R^{D3} are, independently of each other, H or optionally substituted C1-C12 alkyl, with at least one of R^{A2}, R^{A3}, R^{B2}, R^{B3}, R^{C2}, R^{C3}, R^{D2}, and R^{D3} being H; wherein, if X is C or Si,
      - Y^{A1} and Y^{B1} can be covalently linked to form a 5- or 6-membered ring with X, preferably when a1 = b1 = 1; and/or
      - Y^{C1} and Y^{D1} can be covalently linked to form a 5- or 6-membered ring with X, preferably when c1 = d1 = 1;
   wherein, if X is HC-CH and (O)ₐ₁(O)_{b1}X(O)_{C1}(O)_{d1} is 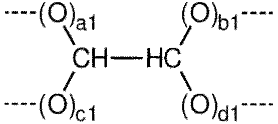
      - Y^{A1} and Y^{C1} can be covalently linked to form a 5- or 6-membered ring with X, preferably when a1 = c1 = 1; and/or
      - Y^{B1} and Y^{D1} can be covalently linked to form a 5- or 6-membered ring with X, preferably when b1 = d1 = 1; or
   wherein, if X is HC-CH and (O)ₐ₁(O)_{b1}X(O)_{C1}(O)_{d1} is 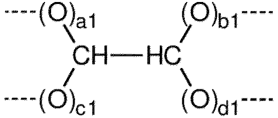
      - Y^{A1} and Y^{B1} can be covalently linked to form a 5- or 6-membered ring with X, preferably when a1 = b1 = 1; and/or
      - Y^{C1} and Y^{D1} can be covalently linked to form a 5- or 6-membered ring with X, preferably when c1 = d1 = 1.

### Definitions

Within the context of the present invention, the term "N-H functionality" is defined as follows: A primary amino group (-NH₂) has two N-H functionalities, a secondary amino group only one N-H functionality, and a tertiary amino group, by consequence, has no reactive N-H functionality.

The term "alkyl" as used herein encompasses both straight and branched alkyl chain radicals and can furthermore also include cycloalkyl radicals. Unless explicitly defined differently, the term "alkyl group" as used herein encompasses groups having up to 12, up to 10, more preferably up to 6 carbon atoms, including, but not being restricted to, methyl, ethyl, propyl, 1-methylethyl, butyl, 1-methylpropyl, 2-methylpropyl, pentyl, 1-methylbutyl, 2-methylbutyl, 3-methylbutyl, 1,1-dimethylpropyl, 1,2-dimethylpropyl, 2,2-dimethylpropyl, and the like. Additionally, the alkyl group is optionally substituted, for example by halogen, preferably fluorine, or cycloalkyls. The term "cycloalkyl radical" as used herein encompasses monocyclic, polycyclic, and spiroalkyl radicals. Preferred cycloalkyl groups are those containing up to 12, more preferably up to 10, more preferably from 3 to 8 ring carbon atoms, including, but not being restricted to, cyclopropyl, cyclopentyl, cyclohexyl, bicyclo[3.1.1]heptyl, spiro[4.5]decyl, spiro[5.5]undecyl, adamantyl, and the like. Further, the cycloalkyl group may be suitably substituted, for example by halogen, deuterium, alkyl or heteroalkyl.

The term "alkylene" as used herein encompasses an alkanediyl functional group, sometimes, but not necessarily, having the free valencies on adjacent carbon atoms.

The term "heteroalkyl group" or "heterocycloalkyl group", if used herein, encompasses an alkyl and a cycloalkyl radical, respectively, having at least one carbon atom replaced by a heteroatom. Unless explicitly defined differently, the term "heteroalkyl group" or "heterocycloalkyl group" as used herein encompasses groups having up to 12, up to 10, more preferably up to 6 carbon atoms. Preferably, the at least one heteroatom is selected from the group consisting of O, S, N, P, B, and Si, and is more preferably O or N. Preferably from 1 to 5, more preferably from 1 to 3, more preferably 1 or 2 heteroatoms may be present in the radical, unless explicitly defined differently herein. The radical can be covalently linked with the remainder of the molecule via a carbon or heteroatom (e.g., N). Further, the heteroalkyl or heterocycloalkyl group may be suitably substituted, for example by halogen, deuterium, alkyl or heteroalkyl.

The term "alkenyl group", if used herein, encompasses both straight and branched chain alkene radicals. Alkenyl groups are essentially alkyl groups with more than one carbon atom that include at least one carbon-carbon double bond in the alkyl chain. The term "cycloalkenyl group", if used herein, encompasses cycloalkyl groups that include at least one carbon-carbon double bond in the cycloalkyl ring. Unless explicitly defined differently, the term "alkenyl group" or "cycloalkenyl group" as used herein encompasses groups having up to 12, preferably up to 10, more preferably up to 6 carbon atoms. Further, the alkenyl group may be suitably substituted, for example by halogen, deuterium, alkyl or heteroalkyl.

The term "heteroalkenyl" if used herein encompasses an alkenyl radical having at least one, preferably from 1 to 5, more preferably 1 to 3, more preferably 1 or 2 carbon atoms replaced by a heteroatom, unless explicitly defined differently herein. Preferably, the at least one heteroatom is selected from the group consisting of O, S, N, P, B, and Si, more preferably O, S, or N. Unless explicitly defined differently, the term "heteroalkenyl group" as used herein encompasses groups having up to 12, preferably up to 10, more preferably up to 6 carbon atoms. Further, the heteroalkenyl group may be suitably substituted, for example by halogen, deuterium, alkyl or heteroalkyl.

The terms "aralkyl" or "arylalkyl", if used herein, are used interchangeably and encompass an alkyl group which is substituted with an aryl group. Further, the aralkyl group may be suitably substituted, for example by halogen, deuterium, alkyl or heteroalkyl.

The term "heterocyclic group" if used herein encompasses aromatic and non-aromatic cyclic radicals containing at least one, preferably 1 to 5, more preferably 1 to 3 heteroatoms. Preferably the at least one heteroatom is selected from the group consisting of O, S, N, P, B, and Si, more preferably is O or N. The term "heteroaromatic cyclic radicals" may be used interchangeably with the term "heteroaryl". Preferred hetero-non-aromatic cyclic groups are those containing from 3 to 7 ring atoms including at least one hetero atom, including, but not being restricted to, cyclic amines such as morpholino, piperidino, pyrrolidino, and the like, and cyclic ethers / thioethers, such as tetrahydrofuran, tetrahydropyran, tetrahydrothiophene, and the like. Further, the heterocyclic group may be optionally substituted, for example by halogen, deuterium, alkyl or aryl. The heterocyclic group can be covalently linked with the remainder of the molecule via carbon and/or heteroatoms, preferably one carbon or nitrogen atom. The heterocyclic group can as well be linked with two carbon and/or heteroatoms with the remainder of the molecule.

The term "aryl group" if used herein encompasses both single-ring aromatic hydrocarbon groups and polycyclic aromatic ring systems. The polycyclic rings may have two or more rings in which two carbons are common to two adjoining rings (the rings are "fused") or wherein one carbon is common to two adjoining rings wherein at least one of the rings is an aromatic hydrocarbon group, e.g., the other rings can be cycloalkyls, cycloalkenyls, aryl, heterocycles, and/or heteroaryls. Preferred aryl groups are those containing 3 to 8 aromatic carbon atoms. Especially preferred is an aryl group having 6 carbons. Suitable aryl groups include phenyl, and radialene. Most preferred is phenyl which is optionally substituted by one or more non-aromatic groups. Further, the aryl group may be substituted, for example by halogen, alkyl, heteroalkyl, or deuterium. The aryl group is connected to the remainder of the molecule via one or two carbon atoms.

The term "heteroaryl" if used herein refers to and includes both single-ring aromatic groups and polycyclic aromatic ring systems that include at least one heteroatom. The heteroatoms include, but are not limited to and are preferably selected from the group consisting of O, S, N, P, B, and Si, and is more preferably O or N. Hetero-single ring aromatic systems are preferably single rings with 5 or 6 ring atoms, and the ring can have from one to five/six, preferably 1 to 3 heteroatoms. The hetero-polycyclic ring systems can have two or more rings in which two atoms are common to two adjoining rings (the rings are "fused") or wherein one carbon is common to two adjoining rings wherein at least one of the rings is a heteroaryl, e.g., the other rings can be cycloalkyls, cycloalkenyls. The hetero-polycyclic aromatic ring systems can have from 1 to 5, preferably 1 to 3 heteroatoms per ring of the polycyclic aromatic ring system. Preferred heteroaryl groups are those containing three to thirty carbon atoms, preferably three to twenty carbon atoms, more preferably three to twelve carbon atoms. Suitable heteroaryl groups include dibenzothiophene, dibenzofuran, dibenzoselenophene, furan, thiophene, benzofuran, benzothiophene, benzoselenophene, carbazole, indolocarbazole, pyridylindole, pyrrolodipyridine, pyrazole, imidazole, triazole, oxazole, thiazole, oxadiazole, oxatriazole, dioxazole, thiadiazole, pyridine, pyrrole, pyrazole, pyridazine, pyrimidine, pyrazine, triazine, oxazine, oxathiazine, oxadiazine, indole, benzimidazole, indazole, indoxazine, benzoxazole, benzisoxazole, benzothiazole, quinoline, isoquinoline, cinnoline, quinazoline, quinoxaline, naphthyridine, phthalazine, pteridine, xanthene, acridine, phenazine, phenothiazine, phenoxazine, benzofuropyridine, furodipyridine, benzothienopyridine, thienodipyridine, benzoselenophenopyridine, and selenophenodipyridine, preferably 1,3,4-oxadiazole, furan, thiophene, 1,2,4-triazole, 1,2,3-triazole, and pyrazole groups, from which one hydrogen atom has been removed from a hydrogen-bearing carbon or heteroatom to form the covalent link to the remainder of the molecule. The heteroaryl group with two hydrogens removed can as well be linked with two carbon and/or heteroatoms with the remainder of the molecules, in which case the heteroaryl group can be part of a larger cyclic group. Further, the heteroaryl group may be suitably substituted, for example by halogen, deuterium, alkyl or aryl.

If one or more of the above-defined groups are substituted, preferred substituents may be selected from the group consisting of deuterium, halogen, alkyl, cycloalkyl, heteroalkyl, heterocycloalkyl, arylalkyl, alkoxy, aryloxy, amino, silyl, alkenyl, cycloalkenyl, heteroalkenyl, alkynyl, aryl, heteroaryl, acyl, carboxylic acid, ether, ester, nitrile, isonitrile, sulfanyl, sulfinyl, sulfonyl, phosphino, and combinations thereof. In some instances, the preferred general substituents may be selected from the group consisting of deuterium, alkyl, cycloalkyl, heteroalkyl, alkoxy, aryloxy, amino, silyl, alkenyl, cycloalkenyl, heteroalkenyl, aryl, heteroaryl, nitrile, isonitrile, sulfanyl, and combinations thereof. In yet other instances, the preferred substituents may be selected from the group consisting of halogen, alkyl, aryl, heteroalkyl, heteroaryl, alkenyl, cycloalkyl, heterocyclic groups, or of deuterium, alkyl, cycloalkyl, aryl, heteroaryl, and combinations thereof. Generally, the terms "substituted" and "substitution" if used herein refer to a substituent other than H that is bonded to the relevant position, for example a carbon atom or a nitrogen atom. For example, when α represents mono-substitution, then one α must be other than H (i.e., a substitution). Similarly, when α represents di-substitution, then two of α must be other than H. Similarly, when α represents no substitution, α, for example, can be a hydrogen for available valencies of straight or branched chain or ring atoms, as in carbon atoms for benzene and the nitrogen atom in pyrrole, or simply represents nothing for ring atoms with fully filled valencies, e.g., the nitrogen atom in pyridine. The maximum number of substitutions possible in a straight or branched chain or ring structure generally depends on the total number of available valencies in the ring atoms or number of hydrogen atoms that can be replaced. All residues and substituents are selected in a way that a chemically stable and accessible chemical group results. Generally, any type of substituent may replace a hydrogen atom in an organic or heterorganic group of compound (I) or (II), as long as it results in a chemical compound which is stable under conditions of steps (a) and (b) and which does not chemically react with other compounds and components of the process.

In some instances, a pair of adjacent residues can be optionally joined (i.e., covalently linked with each other) or fused into a ring. The preferred ring formed therewith is a five-, six-, or seven-membered carbocyclic or heterocyclic ring, including both instances where the portion of the ring formed by the pair of substituents is saturated and where the portion of the ring formed by the pair of substituents is unsaturated. As used herein, "adjacent" means that the two substituents involved can be on the same ring next to each other, or on two neighboring rings having the two closest available substitutable positions, such as 2,2' positions in a biphenyl, or 1,8 position in a naphthalene, or 2,3-positions in a phenyl, or 1,2-positions in a piperidine, as long as they can form a stable fused ring system. In some instances, a pair of non-adjacent substituents can be optionally joined usually by an, at least partial, alkane or heteroalkene chain of atoms, thereby forming another macrocyclic ring system as part of the macrocyclic ring present as part of the coordination compound. In some instances, a pair of substituents present on the same carbon atom can be optionally joined, i.e., into an aryl or heteroaryl group, thus, forming a spiro linkage on said particular carbon atom.

The term "organyl", if used herein, refers to any chemically stable organic arrangement of atoms where one or more hydrogen atom have been removed such as to use those free vacancies to covalently link the organic group with another molecular entity. Thus, the term "organyl" encompasses the majority of the above defined organic groups, for example alkyl, cycloalkyl, heteroalkyl, alkoxy, aryloxy, amino, silyl, alkenyl, cycloalkenyl, heteroalkenyl, aryl, heteroaryl, nitrile, isonitrile, sulfanyl, and combinations thereof with the number of carbon atoms and heteroatoms as defined above for the respective term. The term "organyl" shall especially as well encompass sufficiently chemically stable negatively charged species, such as anions, or anionic side groups.

The terms "halo," "halogen," and "halide", if used herein, are used interchangeably and refer to fluorine, chlorine, bromine, and iodine.

According to the present invention, it is especially preferred that in the compounds (I), (I1) and (II), there are no substituents.

### Process step a)

Preferred features of the process, starting and intermediate compounds and products of the present invention as defined above are as follows:
Preferably, the C2-C4 alkylene oxide is ethylene oxide, 1,2-propylene oxide, 1,2-butylene oxide or a mixture thereof, preferably ethylene oxide, 1,2-propylene oxide or a mixture thereof.

Preferably, the amine of general formula (I) comprises at least two terminal groups NH which are reacted with the alkylene oxide.

Preferably, if a1 = 1, then a2 = 1; if b1 = 1, then b2 = 1; if c1 = 1, then c2 = 1; and if d1 = 1, then d2 = 1.

Preferably,
R^{A2}, R^{B2}, R^{C2} and R^{D2} are H, methyl, ethyl, propyl or isopropyl, more preferably H, and R^{A3}, R^{B3}, R^{C3}, R^{D3} are H, or
R^{A2}, R^{B2}, R^{C2} and R^{D2} are H, methyl, ethyl, propyl or isopropyl, more preferably H, and R^{A3}, R^{B3}, R^{C3}, R^{D3} are methyl, or
R^{A2}, R^{B2}, R^{C2} and R^{D2} are H, methyl, ethyl, propyl or isopropyl, more preferably H, and R^{A3}, R^{B3}, R^{C3}, R^{D3} are ethyl, or
R^{A2}, R^{B2}, R^{C2} and R^{D2} are H, methyl, ethyl, propyl or isopropyl, more preferably H, and R^{A3}, R^{B3}, R^{C3}, R^{D3} are propyl, or
R^{A2}, R^{B2}, R^{C2} and R^{D2} are H, methyl, ethyl, propyl or isopropyl, more preferably H, and R^{A3}, R^{B3}, R^{C3}, R^{D3} are isopropyl.

Preferably, all alkyl groups and alkylene groups present in the at least one amine of general formula (I) are non-branched groups.

Preferably, all alkyl groups and alkylene groups present in the at least one amine of general formula (I) are C1-C6 groups, preferably C1-C4 groups.

Preferably, if X is C, two, one or none of Y^{A1}, Y^{B1}, Y^{C1} and Y^{D1} are H, more preferably wherein one or none of Y^{A1}, Y^{B1}, Y^{C1} and Y^{D1} is H, more preferably wherein none of Y^{A1}, Y^{B1}, Y^{C1} and Y^{D1} is H.

Preferably, if X is Si or HC-CH, none of Y^{A1}, Y^{B1}, Y^{C1} and Y^{D1} is H.

Preferably, X is C and a1 + b1 + c1 + d1 = 2.

Preferably, X is Si and a1 + b1 + c1 + d1 ≥ 2.

Preferably, X is HC-CH and a1 + b1 + c1 + d1 = 2 or a1 + b1 + c1 + d1 = 4.

Preferably, the at least one amine of general formula (I) does not contain hydroxyl groups and/or aromatic groups.

Preferably, in the at least one amine of general formula (I) one or more amino groups are NH₂ groups that preferably are terminal NH₂ groups, and/or the at least one amine of general formula (I) contains 2 to 4 amino groups.

Preferably, the at least one amine of general formula (I), the at least one intermediate (I1) and the at least one polyamine compound (II) do not contain halogen and sulfur atoms, do not contain aromatic groups and do contain at most two heterocyclic groups.

Preferably, the at least one amine of general formula (I), the at least one intermediate (I1) and the at least one polyamine compound (II) are formed solely of C, H, O, N, and optionally additionally one or two, preferably one, Si atoms.

Preferably, when X is C, then the at least one amine of general formula (I), the at least one intermediate (11) and the at least one polyamine compound (II) have a molecular weight of at most 480 g/mol, more preferably of at most 460 g/mol, more preferably of at most 440 g/mol.

Generally, the amine of general formula (I) can be any one of the respective compounds disclosed in WO 2012071896 A1, WO 2013007128 A1, WO 2013184827 A1, WO 201554698 A1, WO 2019240840 A1, WO 2019240841 A1, US 20190016667 A1, US 20190016870 A1, US 20220356145 A1 or US 20220024854 A1, provided that it exhibits a structure according to formula (I) of the present invention.

Preferably, step a) is carried out at a temperature in the range of from 5 to 200 °C, more preferably at a temperature in the range of from 30 to 180 °C, more preferably at a temperature in the range of from 50 to 150 °C, and/or step a) is carried out at a pressure in the range of from 1 to 200 bar(abs), more preferably at a pressure in the range of from 1 to 150 bar(abs), more preferably at a pressure in the range of from 1 to 100 bar(abs).

Preferably, step a) is carried out in the presence of an alkoxylation catalyst, wherein the alkoxylation catalyst is preferably selected from the group consisting of water and heterogeneous catalysts, more preferably selected from the group consisting of water, ion exchange resins and zeolites, the zeolites optionally being doped with one or more rare-earth elements, wherein most preferably, the catalyst is water.

Preferably, step a) is carried out at a molar ratio of alkylene oxide relative to the amino groups of the at least one amine of general formula (I) of from 0.50 to 1.50, more preferably of from 0.75 to 1.25, more preferably of from 0.90 to 1.10.

### Process step b)

Preferably, step b) is carried out at a temperature in the range of from 150 to 260 °C, more preferably in the range of from 170 to 240 °C, more preferably in the range of from 190 to 220 °C, and/or step b) is carried out at a pressure in the range of from 1 to 325 bar(abs), more preferably in the range of from 20 to 220 bar(abs), more preferably at a pressure in the range of from 60 to 190 bar(abs).

Preferably, in step b) the at least one first intermediate (!1) and the one or more of ammonia and at least one amine HNR'R" are employed at a molar ratio of the one or more of ammonia and at least one amine HNR'R" relative to the hydroxyl groups in the at least one first intermediate (I1) in the range of from 0.1 to 50.0, preferably in the range of from 0.5 to 40.0, more preferably in the range of from 1.0 to 30.0. It is conceivable that the molar amount of the ammonia and amine HNR'R" exceeds the molar amount of hydroxyl groups in the intermediate (!1) at the start of the reaction in a quantity of 5 to 5000 mol-%, preferably 50-1000 mol-%, based on the amount of hydroxyl groups in the intermediate (11).

The catalyst used for this type of amination according to step b) can be a homogeneous catalyst and is preferably a heterogeneous catalyst which, more preferably, comprises one or more of copper, nickel and cobalt, optionally together with one or more of rhodium, iridium, palladium, platinum, silver and/ gold, optionally with one or more of iron, manganese, ruthenium, rhenium, molybdenum, tin, and lanthanum. More preferably, the heterogeneous catalyst comprises one or more of copper, nickel and cobalt, more preferably comprises copper, nickel and cobalt. More preferably, the heterogeneous catalyst additionally comprises
- either oxygen compounds of aluminum, of copper, of nickel and of cobalt, and, preferably in the range from 0.2 to 5.0 weight-%, of oxygen compounds of tin, calculated as SnO, previous to the activation of the catalyst by hydrogen;
- or oxygen compounds of aluminum, of copper, of nickel, of cobalt and of tin, and preferably in the range from 0.2 to 5.0 weight-%, of oxygen compounds of one or more of yttrium, lanthanum, cerium and hafnium, calculated as Y₂O₃, La₂O₃, Ce₂O₃ or Hf₂O₃, respectively, previous to the activation of the catalyst by hydrogen.

In a preferred embodiment, a catalyst according to the catalysts claimed in WO 2011/067199 is used, particularly a catalyst according to WO 2011/067199, example 5.

Generally, the catalyst load can be varied in the range of from 0.01 to 2 kg/L/h, preferably in the range of from 0.1 to 1.0 kg/L/h, more preferably in the range of from 0.2 to 0.8 kg/L/h.

The reaction may be carried out using stirred tank reactors, fixed-bed tube reactors and multitube reactors. It may be carried out in batch, semi-batch and continuous mode and with and without recycling of the crude reaction mixture. In a preferred embodiment, the reaction is carried out in continuous mode in a fixed-bed tube reactor.

Compound (I) and preferably compound (II) may comprise at least one NH group or functionality which is preferably a NH₂ group or functionality but can also be a substituted group or functionality NHR. An N-H functionality in compound (II) makes the compound especially suitable for use in epoxy resins, as outlined below. Further preferably, if the alcohol amination according to the present invention is carried out in the presence of a secondary amine, such as dimethyl amine, a compound (II) which contains exclusively tertiary amino groups is obtained; such a compound (II) can be used advantageously as a PU catalyst as described herein.

Polyamine compound (II) may preferably contain primary amino groups, as shown above, and can additionally contain, e.g. via backbone substitution, -NR- or -NH-.

For amination of compound (I1), HNR'R", single compounds or mixtures can be employed.

In step b), in addition to hydrogen, ammonia, primary amines and/or secondary amines can be employed.

### Preferred work-up

Preferably, the process comprises subjecting the reaction mixture obtained from the amination of at least one intermediate (!1) to a solid-liquid separation stage, preferably a filtration stage, wherein the catalyst and, if present, fines are separated, obtaining a liquid mixture being depleted in catalyst and optionally fines.

Preferably, the process further comprises subjecting the liquid mixture being depleted in catalyst and optionally fines to further purification, said purification preferably comprising a distillation stage.

Preferably, ammonia is used as amination agent for carrying out the amination and preferably the process comprises separating excess ammonia from the obtained amination reaction mixture, preferably by distillation as defined above, wherein more preferably, at least a part of said separated ammonia is recycled back as amination agent.

In a preferred embodiment, excess amine HNR'R" is separated from the product of the reaction by one-step distillation. The term "one-step distillation" is understood to mean a distillation with only a single separating stage, as is the case in a simple distillation setup where vapor generated in a reboiler is immediately channeled into a condenser. Contrarily, rectification columns, e.g., have several separating stages and represent a fractional distillation. Preferably, the separated excess amine HNR'R" is recycled to the further production of the amine (II).

It is to be understood that when a molecular fragment is described as being a substituent or otherwise attached to another moiety, its name may be written as if it were a fragment (e.g., phenyl, phenylene, naphthyl, dibenzofuryl) or as if it were the whole molecule (e.g., benzene, naphthalene, dibenzofuran). As used herein, these different ways of designating a substituent or attached fragment are considered to be equivalent.

All individual steps of the process of the present invention can be carried out continuously, in batch mode or in semi-batch mode.

### Exemplary compounds

The structural unit X is C or Si or HC-CH, leading to the following exemplary structures of compound (I). Compound (I) can be obtained by amination of the following hydroxyl compounds with preferably ammonia or suitable amines for this reaction including monomethylamine (MMA), monoethylamine, monopropylamine and monoisopropylamine as well as the respective secondary amines like dimethylamine (DMA):

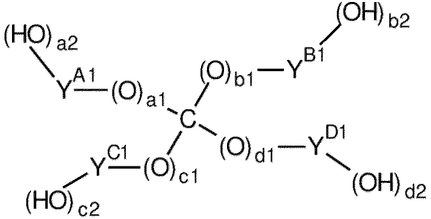

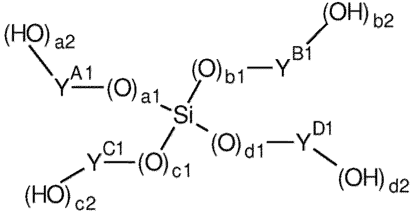

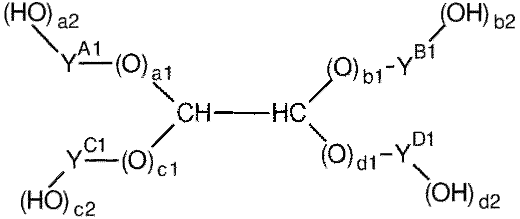

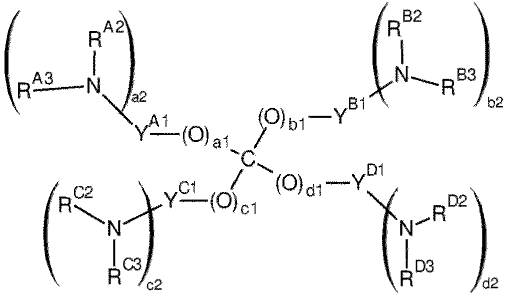

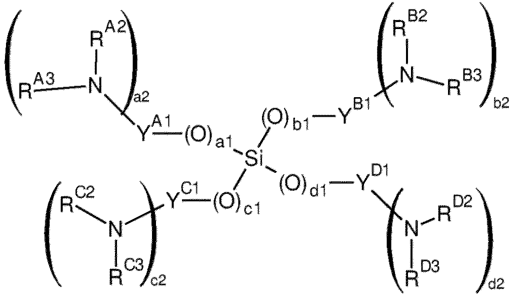

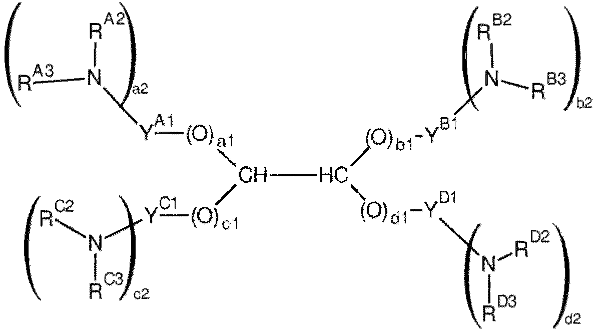

The following exemplary compounds (II) (and respective intermediates (!1)) are also mentioned: 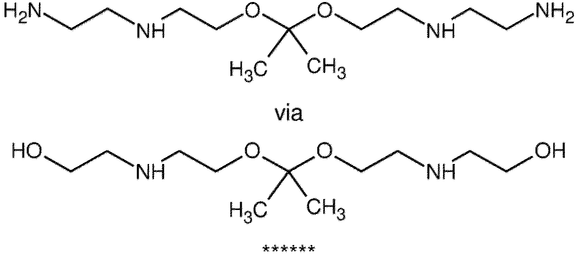 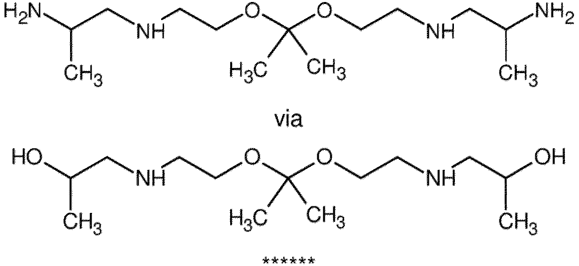 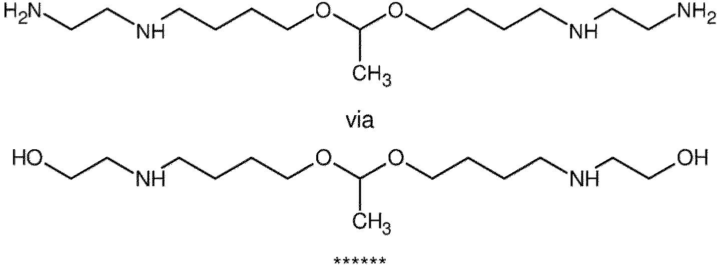 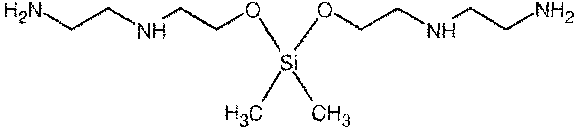 via 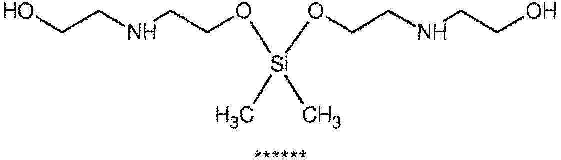 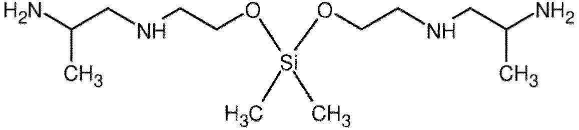 via 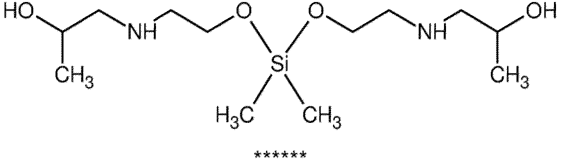 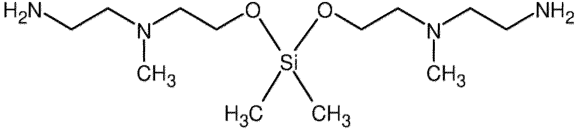 via 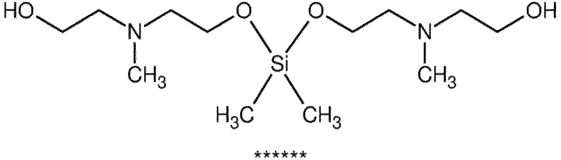 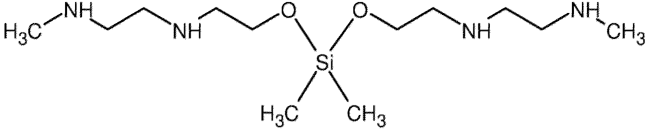 via 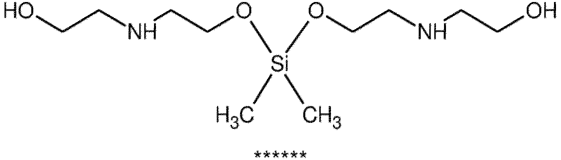 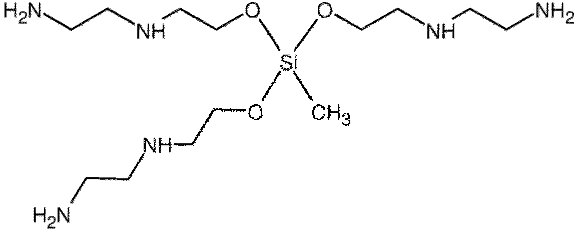 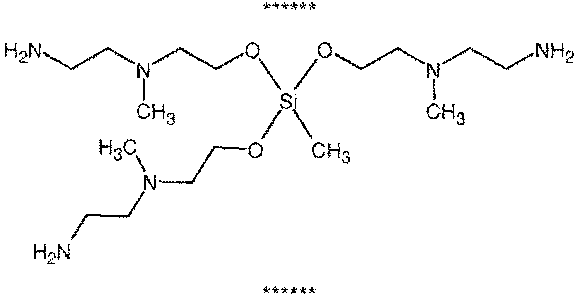 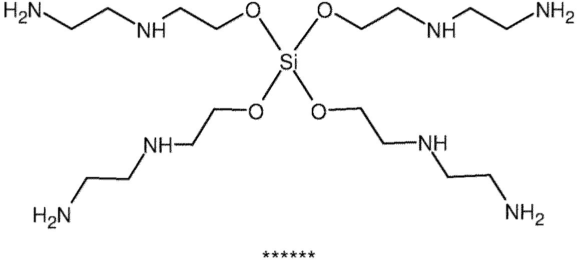 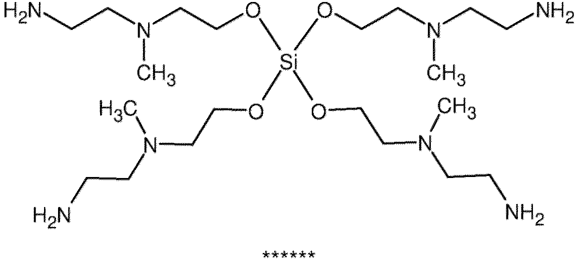 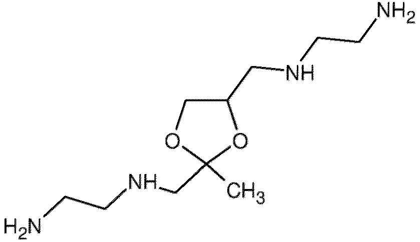 via 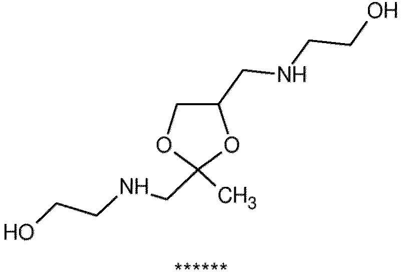 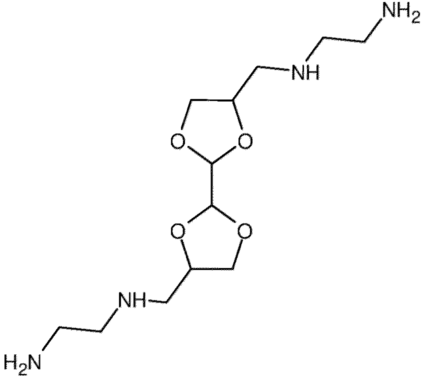 via 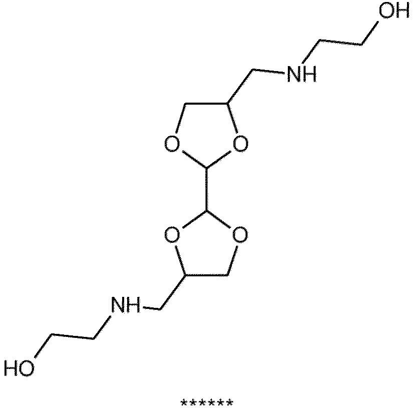 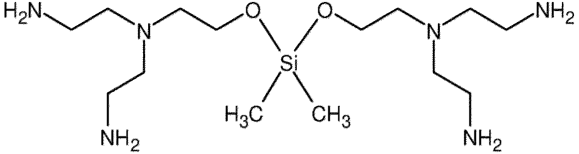 via

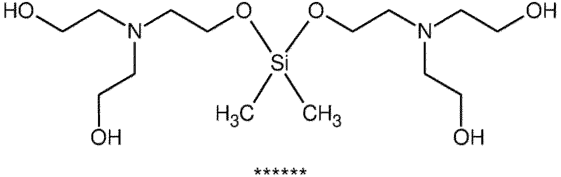

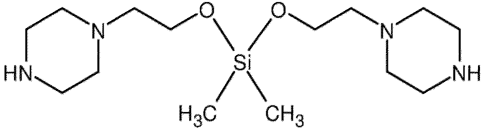

Preferably, the amine of general formula (I) comprises at least two, preferably terminal, groups NH which are reacted with the alkylene oxide.

### Compounds and uses

Further, the present invention relates to an intermediate (!1), obtainable or obtained by the above process.

The present invention also relates to an intermediate (11), wherein at least one, preferably terminal, group NH of the amine of general formula (I) is converted to a group N-CH₂-CHR-OH or N-CHR-CH₂-OH with R being H or methyl or ethyl, preferably N-CH₂-CH₂-OH, as defined above, wherein in said intermediate (!1) preferably at least two, preferably terminal, groups NH of the amine of general formula (I) are converted to groups N-CH₂-CHR-OH or N-CHR-CH₂-OH with R being H or methyl or ethyl, preferably N-CH₂-CH₂-OH.

Further, the present invention relates to a polyamine compound (II), obtainable or obtained by the above process.

Further, the present invention relates to a polyamine compound (II) comprising at least one, preferably terminal, group NH, as defined above, preferably comprising at least two, preferably terminal, groups NH.

The present invention also relates to the use of a polyamine compound (II) as defined above in or as co-reactant, hardener or curative for epoxy resins.

Further, the present invention relates to the use of a polyamine compound (II) as defined above which contains at least one primary or secondary amine group or of a polyamine compound (II) as defined above in or as co-reactant, hardener or curative for epoxy resins.

The present invention also relates to an epoxy resin composition, containing a polyamine compound (II) as defined above, preferably as co-reactant, hardener or curative.

Further, the present invention relates to an epoxy resin composition, containing a polyamine compound (II) as defined above, which contains at least one primary or secondary amine group or containing a polyamine compound (II) as defined above, preferably as a co-reactant, a hardener or a curative.

The present invention also relates to an epoxy resin composition, containing a polyamine compound (II) as defined above, reacted as co-reactant, hardener or curative with an epoxy resin.

Further, the present invention relates to an epoxy resin composition, containing a polyamine compound (II) as defined above, which contains at least one primary or secondary amine group or a polyamine compound (II) as defined above, reacted as a co-reactant, a hardener or a curative with an epoxy resin.

Further, the present invention relates to the use of a polyamine compound (II) as defined above, or of compounds prepared therewith, for flotation; corrosion inhibition; asphalt emulsification; fertilizer-anticaking agents; additives for preventing caking of powdered minerals; emulsifiers, adjuvants, and intermediates in pesticide production; additives in plastic formulations; pigment-grinding aids; dispersants for pigments in paints, coatings, and magnetic tape; thickening agents in drilling muds, oil-based coatings and the paint industry; biocidal applications in households, swimming pools and oil fields; lubricants; petroleum additives; deicers for motor fuels; catalysis (such as in polyurethane formation); manufacturing of polyurethanes; intermediates for dyes, and of a polyamine compound (II) as defined above which contains tertiary amino groups as or for producing cleavable PU catalysts that can optionally be incorporated due to additional functional groups or upon cleavage.

The functionalized cleavable amines which result from the functionalization of cleavable amines described herein exhibit a variety of uses. In general, the functionalized cleavable amines can be applied in any application that is typical for amines, as long as the backbone (ketal, acetal or silylketal functionality) does not have an (negative) influence on the application. Examples, possibly after further functionalization, include: epoxy hardening; flotation; corrosion inhibition; asphalt emulsification; fertilizer-anticaking agents; additives for preventing caking of powdered minerals; emulsifiers, adjuvants, and intermediates in pesticide production; additives in plastic formulations; pigment-grinding aids; dispersants for pigments in paints, coatings, and magnetic tape; thickening agents in drilling muds, oil-based coatings and the paint industry; biocidal applications in households, swimming pools and oil fields; lubricants; petroleum additives; deicers for motor fuels; catalysis (such as in polyurethane formation); manufacturing of polyurethanes; intermediates for dyes.

### Biodegradability

As mentioned above, cleavable amines can potentially provide a solution to increase the recyclability of epoxy systems or enhance the biodegradability of polyamine-based polymers.

The use of cleavable amines as epoxy hardeners enables better recycling of resulting epoxy systems, or as starting materials for polymers to increase the biodegradability.

Ideally, the cleavable amines are readily biodegradable, i.e., show equal to or more than 60% oxygen consumption within 28 days in the OECD 301 F test or at least show equal to or more than 60% within 56 days in the OECD 301 F test. Alternatively, the cleavable amines are inherently biodegradable in the OECD 302 B test, i.e., show equal to or more than 70% dissolved organic carbon (DOC) levels. Even an incremental improvement of the biodegradability to more than 20%, preferably more than 40% and even more preferably to more than 50% oxygen consumption within 28 days in the OECD 301 F test would be a technical advantage vs. the current state of the art (no biodegradability at all or less than 10% oxygen consumption within 28 days in the OECD 301 F test). Hence, the present invention addresses the need to find improved cleavable amine architectures with a superior performance profile, a feasible preparation process and an improved biodegradation behavior.

Preferably, compounds (I) and (II) demonstrate at least 20%, preferably at least 40% or more preferably at least 60% biodegradability according to the OECD 301 A, B, C, D, E or F or OECD 302 B, preferably OECD 301 F, standard within 56 days, preferably within 28 days.

In preferred embodiments, compounds (I) and (II) of the present invention demonstrate a biodegradation of at least 20%, at least 30%, at least 40%, at least 50%, or at least 60% biodegradability according to the OECD standard 301 B, C, D or F within 28 days. The OECD standards 301 B, C, D and F are all respirometry test methods. The skilled person is well-aware of these tests and therefore solely OECD standard 301 F will be described representatively.

For the purposes of this invention, aerobic biodegradation in wastewater according to OECD 301 F is expressed as a percentage of the theoretical oxygen demand (ThOD, which is measured by the elemental analysis of the compound of interest), which is needed to completely biodegrade the compound sample. Thus, the amount of oxygen taken up by the microbial population during biodegradation of the test substance (corrected for uptake by blank inoculum, run in parallel) is expressed as a percentage of ThOD. The obtained values are preferably measured in triplicate using the OECD 301 F manometric respirometry method. The consumption of oxygen is determined by measuring the change in pressure in the apparatus using an OxiTop^{®} C (Xylem 35 Analytics Germany Sales GmbH & Co KG). Details for the tests performed are given in the experimental section below.

The OECD standards OECD 301 A, E and 302 B are Dissolved Organic Carbon (DOC) tests. Also, these tests are well-known for the skilled person. Therefore, solely OECD standard 301 A will be described representatively.

Standard OECD 301 A is a DOC Die Away Test: The test substance is used at a high concentration compared to the other tests, i.e. 10-40 mgDOC/L (DOC = Dissolved Organic Carbon). The DOC concentration is measured at defined intervals over a period of 28 days.

However, the skilled person is also aware of other tests that can be used to determine the biodegradability of the inventive compounds. These tests include chemical, physical and biological tests. For example, the used test may be based on the specific activity of an indicator enzyme or the amount of expression of an indicator gene. Physical tests can involve physical parameters, such as the density, surface tension or electric conductivity of the solvent of the sample. In such tests, the inventive compounds demonstrate a significant biodegradability, i.e. at least 20%, at least 30%, at least 40%, at least 50%, at least 60% or at least 70% of the maximum biodegradation.

In preferred embodiments, compounds (I) and (II) of the present invention demonstrate a biodegradation of at least 20%, at least 30%, at least 40%, at least 50%, at least 60% or at least 70% biodegradability according to the OECD standard 301 A, E or 302 B within 28 days, and/or demonstrate at least 20%, preferably at least 40% or more preferably at least 60% biodegradability according to the OECD 301 A, B, C, D, E or For OECD 302 B, preferably OECD 301 F, standard within 56 days, preferably within 28 days.

### Further aspect

According to another aspect, the process of the present invention comprises the step of converting a chemical material obtainable by or obtained by the process as defined above to obtain a product Ω.

Preferably, the product Ω is selected from:
- building block or monomer; or
- polymer, preferably polymer A, polymer composition, preferably polymer composition A, or polymer product, preferably polymer product A; or
- agrochemical composition, agrochemical formulation auxiliary or agrochemically active ingredient; or
- active pharmaceutical ingredient or intermediate thereof, pharmaceutical excipient, animal feed additive, human food additive, dietary supplements, aroma chemical or aroma composition; or
- aqueous polymer dispersion, preferably polyurethane or polyurethane - poly(meth)acrylate hybrid polymer dispersion, emulsion, binder for paper and fiber coatings, UV-curable acrylic polymer for hot melts and coatings polyisocyanates, hyperbranched polyester polyol, polymeric dispersant for inorganic binder compositions, unsaturated polyester polyol or 100% curable composition; or
- cosmetic surfactant, emollient, wax, cosmetic polymer, UV filter, further cosmetic ingredient or composition or formulation thereof; or
- polymer B, polymer composition B, coating composition, other functional composition, foil, molded body, coating or coated substrate.

Preferably, the content of the chemical material in the product Ω is 1 weight-% or more, preferably 2 weight-% or more, more preferably 5 weight-% or more, more preferably 15 weight-% or more, more preferably 30 weight-% or more, more preferably 40 weight-% or more, more preferably 60 weight-% or more, more preferably 80 weight-% or more, more preferably 90 weight-% or more, more preferably 95 weight-% or more; and/or the content of the chemical material in the product Ω is 100 weight-% or less, preferably 95 weight-% or less, more preferably 90 weight-% or less, more preferably 50 weight-% or less, more preferably 25 weight-% or less, more preferably 10 weight-% or less; and preferably wherein the content is determined based on identity preservation and/or segregation and/or mass balance and/or book and claim chain of custody models, preferably based on mass balance, preferably the International Sustainability and Carbon Certification (ISCC) standard.

The publication Prior Art Disclosure; Issue 684; paragraphs [1000] to [8005]; ISSN: 2198-4786; published: February 12, 2024 will be regarded as Reference RF1, which is incorporated herein by reference in its entirety. Preferably, the product Ω is a product as described in Reference RF1; paragraphs [1000] to [8005]. Preferably, the process described herein is further a process for the production of a product.

The converting step to obtain the product Ω preferably comprises one or more step(s) as described below and can be performed by conventional methods well known to a person skilled in the art. The converting step preferably comprises one or more step(s) selected from:
recycling, preferably depolymerizing, gasifying, pyrolyzing, and/or steam cracking; and/or purifying, preferably crystallizing, (solvent) extracting, distilling, evaporating, hydrotreating, absorbing, adsorbing and/or subjecting to ion exchanger; and/or
assembling, preferably foaming, synthesizing, chemical conversion, chemically transforming, polymerizing and/or compounding; and/or
forming, preferably foaming, extruding and/or molding; and/or
finishing, preferably coating and/or smoothing.

In addition, the one or more step(s) are described in detail in Reference RF1; paragraphs [1000] to [8005].

The term "building block", as used in the context of the product Ω herein, comprises compounds, which are in a gaseous or liquid state under standard conditions of 0°C and 0.1 MPa. Building blocks are typically used in chemical industry to form secondary products, which provide a higher structural complexity and/or higher molecular weight than the building block on which the secondary product is based. The building block is preferably selected from the group consisting of hydrogen, carbon monoxide, carbon dioxide, ethylene oxide, ethylene glycols, syngas comprising a mixture of hydrogen and carbon monoxide, alkanes, alkenes, alkynes and aromatic compounds. The alkanes, alkenes, alkynes and aromatic compounds comprise in particular 1 to 12 carbon atoms, respectively.

The term "monomer", as used in the context of the product Ω herein, comprises molecules, which can react with each other to form polymer chains by polymerization. The monomer is preferably selected from the group consisting of (meth)acrylic acid, salts of (meth)acrylic acid; in particular sodium, potassium and zinc salts; (meth)acrolein and (meth)acrylates. (Meth)acrylates comprising 1 to 22 carbon atoms are preferred, in particular comprising 1 to 8 carbon atoms. The terms (meth)acrylic acid, (meth)acrolein or (meth)acrylate relate to acrylic acid, acrolein or acrylate and also to methacrylic acid, methacrolein or methacrylate, where applicable. Further, the monomer can be selected from hexamethylenediamine (HMD) and adipic acid.

The building block can further be an intermediate compound. The term "intermediate compound", as used in the context of the product Ω herein, comprises organic reagents, which are applied for formation of compounds with higher molecular complexity. The intermediate compound can be selected for example from the group consisting of phosgene, polyisocyanates and propylene oxide. The polyisocyanates are in particular aromatic di- and polyisocyanates, preferably toluene diisocyanate (TDI) and/or diphenylmethane diisocyanate (MDI).

The building block and the monomer and typical converting step(s) to obtain the building block or monomer are described in more detail in paragraphs [1000] to [1012] of Reference RF1.

The term "polymer A", as used in the context of the product Ω herein, comprises thermoplastic, e.g., polyamide or thermoplastic polyurethane, thermoset, e.g., polyurethane, elastomer, e.g., polybutadiene, or a copolymer or a mixture thereof and is defined in more detail in paragraphs [2001] to [2007] of Reference RF1. The term "polymer composition A", as used in the context of the product Ω herein, comprises all compositions comprising a polymer as described above and one or more additive(s), e.g. reinforcement, colorant, modifier and/or flame retardant, and is defined in more detail in paragraph [2008] of Reference RF1. The term "polymer product A", as used in the context of the product Ω herein, comprises any product comprising the polymer A and/or polymer composition A as described above and is defined in more detail in paragraphs [2009] and [2010] of Reference RF1. The step(s) to obtain the polymer, preferably polymer A, polymer composition, preferably polymer composition A or polymer product, preferably polymer product A is/are described in more detail in paragraph [2011] of Reference RF1.

The term "agrochemical composition", as used in the context of the product Ω herein, typically relates to a composition comprising an agrochemically active ingredient and at least one agrochemical formulation auxiliary. Examples of agrochemical compositions, active ingredients and auxiliaries are described in more detail in Reference RF1, paragraph [4001]. The agrochemical composition may take the form of any customary formulation. The agrochemical compositions are prepared in a known manner, e.g. described by Mollet and Grubemann, Formulation technology, Wiley VCH, Weinheim, 2001; or Knowles, New developments in crop protection product formulation, Agrow Reports DS243, T&F Informa, London, 2005. The converting step(s) to obtain the agrochemically active ingredients and auxiliaries may be conducted in analogy to the production step(s) of their analogues that are based on petrochemicals or other precursors that are not gained by recycling processes. In addition, conversion to compounds mentioned in sections "Polymer" and "Cosmetic surfactant, emollient, wax, cosmetic polymer, UV filter, further cosmetic ingredient or compositions or formulations thereof" may be performed as described in these sections as well as the respective paragraphs in Reference RF1.

The term active pharmaceutical ingredients and/or intermediates thereof, as used in the context of the product Ω herein, comprises substances that provide pharmacological activity or other direct effect in the diagnosis, cure, mitigation, treatment, or prevention of disease, or to affect the structure or any function of the body. Intermediates thereof are isolated products that are generated during a multi-step route of synthesis of an active pharmaceutical ingredient. The term pharmaceutical excipients, as used in the context of the product Ω herein, comprises compounds or compound mixtures used in compositions for various pharmaceutical applications, which are not substantially pharmaceutically active on itself. Active pharmaceutical ingredients and/or intermediates thereof and pharmaceutical excipients are defined in more detail in paragraph [5001] of Reference RF1. The converting step(s) to obtain the active pharmaceutical ingredients and/or intermediates thereof and pharmaceutical excipients may comprise one or more synthesis steps and can be performed by conventional synthesis and techniques well known to a person skilled in the art.

The terms animal feed additives, human food additives, dietary supplements, as used in the context of the product Ω herein, comprises Vitamins, Pro-Vitamins and active metabolites thereof including intermediates and precursors, especially Vitamin A, B, E, D, K and esters thereof, like acetate, propionate, palmitate esters or alcohols thereof like retinol or salts thereof and any combinations thereof; Tetraterpenes, especially isoprenoids like carotenoids and xanthophylls including their intermediates and precursors as well as mixtures and derivates thereof, especially beta carotene, Canthaxanthin, Citranaxanthin, Astaxanthin, Zeaxanthin, Lutein, Lycopene, Apo-carotenoids, and any combinations thereof; organic acids, especially formic acid, propionic acid and salts thereof, such as sodium, calcium or ammonium salts, and any combinations thereof, such as but not limited to mixtures of formic acid and sodium formiate, propionic acid and ammonium propionate, formic acid and propionic acid, formic acid and sodium formiate and propionic acid, propionic acid and sodium propionate and formic acid and sodium formiate; glycerides of carboxylic acids and short and medium chain fatty acids, conjugated linoleic acids, such as omega-6 fatty acid (C18:2) methyl ester and 1,2-propandiol and beverage stabilizers, such as polyvinylpyrrolidone-polymer or polyvinylimidazole / polyvinylpyrrolidone-copolymer. Animal feed additives, human food additives and dietary supplements are defined in more detail in paragraph [5002] of Reference RF1. The converting step(s) to obtain the animal feed additives, human food additives, dietary supplements may comprise one or more synthesis steps and can be performed by conventional synthesis and techniques well known to a person skilled in the art.

The terms aroma chemical and aroma composition as used in the context of the product Ω herein, comprise a volatile organic substance with a molecular weight between 70-250 g/mol comprising a functional group with a carbon skeleton of C₅-C₁₆ carbon atoms comprising linear, branched, cyclic, for example with a ring size of C₅-C₁₈, bicyclic or tricyclic aliphatic chains and but not necessarily one or more unsaturated structural elements like double bonds, triple bonds, aromatics or heteroaromatics and preferably the one or more additional functional groups are selected from alcohol, ether, ester, ketone, aldehyde, acetal, carboxylic acid, nitrile, thiol, amine. In one aspect, the aroma chemical is a terpene-based aroma chemical, for example selected from monoterpenes and monoterpenoids, sesquiterpenes and sesquiterpenoids, diterpenes, triterpenes or tetraterpenes. Aroma chemicals can be combined with further aroma chemicals to give an aroma composition. Aroma chemicals and aroma compositions are defined in more detail in paragraph [5003] of Reference RF1. The converting step(s) to obtain the aroma chemical and aroma composition may comprise one or more synthesis steps and can be performed by conventional synthesis and techniques well known to a person skilled in the art.

The term "aqueous polymer dispersion", as used in the context of the product Ω herein, comprises aqueous composition(s) comprising dispersed polymer(s) and is defined in more detail in the section [6001] entitled "aqueous polymer dispersion" of Reference RF1. The dispersed polymer(s) may be selected from acrylic emulsion polymer(s), styrene acrylic emulsion polymer(s), styrene butadiene dispersion(s), aqueous dispersion(s) comprising composite particles, acrylate alkyd hybrid dispersion(s), polyurethane(s) (including UV-curable polyurethanes) and polyurethane - poly(meth)acrylate hybrid polymer(s). The term "emulsion polymer", as used in the context of the product Ω herein, comprises polymer(s) made by free-radical emulsion polymerization. Aqueous polyurethane dispersion(s) are defined in more detail in the section [6002] entitled "Polyurethane dispersions" of Reference RF1. UV-curable polyurethane(s) is/are defined in more detail in the section [6017] of Reference RF1. Polyurethane - poly(meth)acrylate hybrid polymer(s) is/are defined in more detail in the section [6016] of Reference RF1.

The term "polymeric dispersant", as used in the context of the product Ω herein, comprises preferably polymer(s) comprising polyether side chain, in particular polycarboxylate ether polymer(s) and polycondensation product(s) defined in more detail in paragraph [6020] entitled "Polymeric dispersant" of Reference RF1.

The converting (polymerization) step(s) to obtain the aqueous polymer dispersion(s) comprising emulsion polymer(s) is/are defined in more detail in the section [6003] entitled "Emulsion polymerization" of Reference RF1.

The converting (polymerization) step(s) to obtain the aqueous polyurethane dispersion(s) is/are defined in more detail in the section [6014] entitled "Process for the preparation of aqueous polyurethane dispersions" and section [6017)] entitled "Aqueous UV-curable polyurethane dispersions, their preparation and use and compositions containing them" of Reference RF1.

Composition(s) and uses of aqueous polymer dispersion(s) and of polymeric dispersant(s) are defined in more detail in the following sections of Reference RF1:
section [6004] entitled "Uses of aqueous polymer dispersions",
section [6005] entitled "Binders for architectural and construction coatings"
section [6006] entitled "Binders for paper coating"
section [6007] entitled "Binders for fiber bonding"
section [6008] entitled "Adhesive polymers and adhesive compositions"
section [6015] entitled "Aqueous polyurethane dispersions suitable for use in coating compositions"
section [6016] entitled "Aqueous polyurethane - poly(meth)acrylate hybride polymer dispersions suitable for use in coating compositions"
section [6017] entitled "Aqueous UV-curable polyurethane dispersions, their preparation and use and compositions containing them"
section [6018] entitled "Inorganic binder compositions comprising polymeric dispersants and their use" [6019] 100% curable coating compositions

UV-crosslinkable poly(meth)acrylate(s) and its/their uses are defined in more detail in section [6009] entitled "UV-crosslinkable poly(meth)acrylates for use in UV-curable solvent-free hotmelt adhesives and their use for making pressure-sensitive self-adhesive articles" of Reference RF1.

Polyisocyanate(s), composition(s) comprising them and their uses are defined in more detail in section [6010] entitled "Polyisocyanates" of Reference RF1.

Hyperbranched polyester polyol(s) and its/their uses are defined in more detail in section [6011] entitled "Organic solvent based hyperbranched polyester polyols suitable for use in coating compositions" of Reference RF1. The converting step(s) to obtain the hyperbranched polyester polyols is/are defined in more detail in the section [6012] entitled "Preparation of organic solvent based hyperbranched polyester polyols" of Reference RF1. Coating composition(s) comprising hyperbranched polyester polyol(s), polyisocyanate(s) and additive(s) and substrate(s) coated therewith are defined in more detail in section [6013] entitled "Organic solvent based two component coating compositions comprising hyperbranched polyester polyols and polyisocyanates" of Reference RF1.

Unsaturated polyester polyol(s), solvent-based coating composition(s) comprising said unsaturated polyester polyol(s) and substrate(s) for coating with said coating composition(s) are defined in more detail in section [6018] entitled "Organic solvent based coating composition comprising unsaturated polyester polyols" of Reference RF1.

100% curable coating composition(s) is/are defined in more detail in section [6019] of Reference RF1.

Polymeric dispersant(s) for inorganic binder compositions is/are defined in more detail in section [6020] of Reference RF1. The inorganic binder composition(s) comprising the polymeric dispersants and their use are defined in more detail in section [6021] of Reference RF1. The converting step(s) to obtain the polymeric dispersant(s) are defined in more detail in section [6020] of Reference RF1. The term "inorganic binder composition" comprising the polymeric dispersant(s), as used in the context of the product Ω herein, comprises preferably in particular hydraulically setting compositions and compositions comprising calcium sulfate and is defined in more detail in section [6021] of Reference RF1 entitled "Inorganic binder compositions comprising the polymeric dispersant and their use". Specific building material formulation(s) comprising polymeric dispersant(s) or building product(s) produced by a building material formulation comprising a polymeric dispersant are disclosed in more detail in section [6021] of Reference RF1.

The term "cosmetic surfactant", as used in the context of the product Ω herein, comprises nonionic, anionic, cationic and amphoteric surfactants and is defined in more detail in paragraph [7002] of Reference RF1. The term "emollient", as used in the context of the product Ω herein, refers to a chemical compound used for protecting, moisturizing, and/or lubricating the skin and is defined in more detail in paragraph [7003] of Reference RF1. The term "wax", as used in the context of the product Ω herein, comprises pearlizers and opacifiers and is defined in more detail in paragraph [7004] of Reference RF1. The term "cosmetic polymer", as used in the context of the product Ω herein, comprises any polymer that can be used as an ingredient in a cosmetic formulation and is defined in more detail in paragraph [7005] of Reference RF1. The term "UV filter", as used in the context of the product Ω herein, refers to a chemical compound that blocks or absorbs ultraviolet light and is defined in more detail in paragraph [7006] of Reference RF1. The term "further cosmetic ingredient", as used in the context of the product Ω herein, comprises any ingredient suitable for making a cosmetic formulation. Several sources disclose cosmetically acceptable ingredients. E. g. the database Cosing on the internet pages of the European Commission discloses cosmetic ingredients and the International Cosmetic Ingredient Dictionary and Handbook, edited by the Personal Care Products Council (PCPC), discloses cosmetic ingredients. The term "composition and/or formulation thereof" with reference to the cosmetic surfactant, emollient, wax, cosmetic polymer, UV filter and/or further cosmetic ingredient refers to personal care and/or cosmetic compositions or formulations defined in more detail in paragraph [7007] of Reference RF1. The converting step(s) to obtain the cosmetic surfactant, emollient, wax, cosmetic polymer, UV filter or further cosmetic ingredient is/are defined in more detail in paragraph [7008] of Reference RF1.

The terms "polymer B", "polymer composition B", "coating composition", "other functional composition", "foil", "molded body", "coating" and "coated substrate" are well known to the person skilled in the art and are defined in more detail from paragraph [8000] to [8005] of Reference RF1.

### Embodiments

The present invention is further illustrated by the following set of embodiments and combinations of embodiments resulting from the dependencies and back-references as indicated. In particular, it is noted that in each instance where a range of embodiments is mentioned, for example in the context of a term such as "The process of any one of embodiments 1 to 4", every embodiment in this range is meant to be explicitly disclosed for the skilled person, i.e. the wording of this term is to be understood by the skilled person as being synonymous to "The process of any one of embodiments 1, 2, 3 and 4". Further, it is explicitly noted that the following set of embodiments represents a suitably structured part of the general description directed to preferred aspects of the present invention, and, thus, suitably supports, but does not represent the claims of the present invention.
1. A process for preparing polyamines via alkylene oxide addition to an amine followed by alcohol amination, comprising
   a) reacting at least one amine of general formula (I) 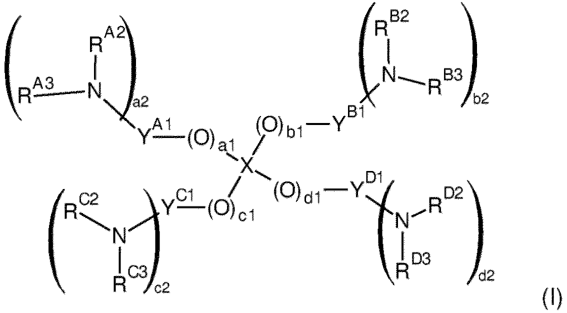 with at least one C2-C4-alkylene oxide, thereby obtaining at least one first intermediate (I1), wherein the amine of general formula (I) comprises at least one, preferably terminal, group NH which is reacted with the C2-C4-alkylene oxide, thereby obtaining at least one first intermediate (I1), wherein at least one, preferably terminal, group NH of the amine of general formula (I) is converted to at least one group N-CH₂-CHR-OH or N-CHR-CH₂-OH with R being H or methyl or ethyl, preferably N-CH₂-CH₂-OH;
   b) aminating the at least one first intermediate (I1) with at least one of ammonia, primary and secondary amines HNR'R" with R' being H, methyl, ethyl, propyl or isopropyl and R" being H, methyl, ethyl, propyl or isopropyl, or mixtures thereof, to form at least one polyamine compound (II), comprising converting the at least one group N-CH₂-CHR-OH or N-CHR-CH₂-OH, preferably the at least one group N-CH₂-CH₂-OH to the at least one group N-CH₂-CHR-NR'R" or N-CHR-CH₂-NR'R", preferably to the at least one group N-CH₂-CH₂-NH₂; and
      if at least one group N(-CH₂-CHR-OH)₂, N(-CHR-CH₂-OH)₂, or N(-CHR-CH₂-OH)(-CH₂-CHR-OH), preferably at least one group N(-CH₂-CH₂-OH)₂, is present in the at least one first intermediate (I1) and at least one of ammonia or primary amines H₂NR' with R' being H, methyl, ethyl, propyl or isopropyl, or mixtures thereof, are employed in step b),
      then step b) further comprises the step of converting the at least one group N(-CH₂-CHR-OH)₂, N(-CHR-CH₂-OH)₂, or N(-CHR-CH₂-OH)(-CH₂-CHR-OH), preferably at least one group N(-CH₂-CH₂-OH)₂, in the at least one first intermediate (!1) with ammonia or primary amine H₂NR' with R' being H, methyl, ethyl, propyl or isopropyl, or mixtures thereof, to form at least one, optionally substituted, piperazine group -N(-CH₂-CHR-)₂NR', -N(-CHR-CH₂-)₂NR' or -N(-CH₂-CHR-)(-CHR-CH₂-)NR', preferably to form at least one piperazine group -N(-CH₂-CH₂-)₂NH;

   wherein X is C or Si or HC-CH;
   wherein a1, a2, b1, b2, c1, c2, d1, d2 are independently of each other 0 or 1, with a1 + b1 + c1 + d1 ≥ 2 and a2 + b2 + c2 + d2 ≥ 2;
   wherein, if a1 = a2 = 0, Y^{A1} is H or optionally substituted C1-C12 alkyl;
   wherein, if a1 = 1 and a2 = 0, Y^{A1} is optionally substituted C1-C12 alkyl;
   wherein, if a1 = a2 = 1, or if a1 = 0 and a2 = 1, Y^{A1} is optionally substituted C1-C12 alkylene;
   wherein if b1 = b2 = 0, Y^{B1} is H or optionally substituted C1-C12 alkyl;
   wherein if b1 = 1 and b2 = 0, Y^{B1} is optionally substituted C1-C12 alkyl;
   wherein if b1 = b2 = 1, or if b1 = 0 and b2 = 1, Y^{B1} is optionally substituted C1-C12 alkylene;
   wherein if c1 = c2 = 0, Y^{C1} is H or optionally substituted C1-C12 alkyl;
   wherein if c1 = 1 and c2 = 0, Y^{C1} is optionally substituted C1-C12 alkyl;
   wherein if c1 = c2 = 1, or if c1 = 0 and c2 = 1, Y^{C1} is optionally substituted C1-C12 alkylene;
   wherein if d1 = d2 = 0, Y^{D1} is H or optionally substituted C1-C12 alkyl;
   wherein if d1 = 1 and d2 = 0, Y^{D1} is optionally substituted C1-C12 alkyl;
   wherein if d1 = d2 = 1, or if d1 = 0 and d2 = 1, Y^{D1} is optionally substituted C1-C12 alkylene;
   wherein R^{A2}, R^{A3}, R^{B2}, R^{B3}, R^{C2}, R^{C3}, R^{D2}, R^{D3} are, independently of each other, H or optionally substituted C1-C12 alkyl, with at least one of R^{A2}, R^{A3}, R^{B2}, R^{B3}, R^{C2}, R^{C3}, R^{D2}, and R^{D3} being H;
   wherein, if X is C or Si,
      - Y^{A1} and Y^{B1} can be covalently linked to form a 5- or 6-membered ring with X, preferably when a1 = b1 = 1; and/or
      - Y^{C1} and Y^{D1} can be covalently linked to form a 5- or 6-membered ring with X, preferably when c1 = d1 = 1;
   wherein, if X is HC-CH and (O)ₐ₁(O)_{b1}X(O)_{c1}(O)_{d1} is 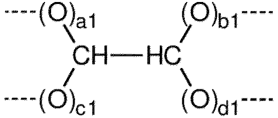
      - Y^{A1} and Y^{C1} can be covalently linked to form a 5- or 6-membered ring with X, preferably when a1 = c1 = 1; and/or
      - Y^{B1} and Y^{D1} can be covalently linked to form a 5- or 6-membered ring with X, preferably when b1 = d1 = 1; or
   wherein, if X is HC-CH and (O)ₐ₁(O)_{b1}X(O)_{c1}(O)_{d1} is 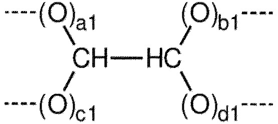
      - Y^{A1} and Y^{B1} can be covalently linked to form a 5- or 6-membered ring with X, preferably when a1 = b1 = 1; and/or
      - Y^{C1} and Y^{D1} can be covalently linked to form a 5- or 6-membered ring with X, preferably when c1 = d1 = 1.
2. The process of embodiment 1, wherein the C2-C4 alkylene oxide is ethylene oxide, 1,2-propylene oxide, 1,2-butylene oxide or a mixture thereof, preferably ethylene oxide, 1,2-propylene oxide or a mixture thereof.
3. The process of embodiment 1 or 2, wherein the amine of general formula (I) comprises at least two, preferably terminal, groups NH which are reacted with the alkylene oxide.
4. The process of any one of embodiments 1 to 3, wherein if a1 = 1, then a2 = 1; if b1 = 1, then b2 = 1; if c1 = 1, then c2 = 1; and if d1 = 1, then d2 = 1.
5. The process of any one of embodiments 1 to 4, wherein R^{A2}, R^{B2}, R^{C2} and R^{D2} are H, methyl, ethyl, propyl or isopropyl, more preferably H, and R^{A3}, R^{B3}, R^{C3}, R^{D3} are H, or wherein R^{A2}, R^{B2}, R^{C2} and R^{D2} are H, methyl, ethyl, propyl or isopropyl, preferably H, and R^{A3}, R^{B3}, R^{C3}, R^{D3} are methyl or wherein R^{A2}, R^{B2}, R^{C2} and R^{D2} are H, methyl, ethyl, propyl or isopropyl, preferably H, and R^{A3}, R^{B3}, R^{C3}, R^{D3} are ethyl, or wherein R^{A2}, R^{B2}, R^{C2} and R^{D2} are H, methyl, ethyl, propyl or isopropyl, preferably H, and R^{A3}, R^{B3}, R^{C3}, R^{D3} are propyl, or wherein R^{A2}, R^{B2}, R^{C2} and R^{D2} are H, methyl, ethyl, propyl or isopropyl, preferably H, and R^{A3}, R^{B3}, R^{C3}, R^{D3} are isopropyl.
6. The process of any one of embodiments 1 to 5, wherein all alkyl groups and alkylene groups present in the at least one amine of general formula (I) are non-branched groups.
7. The process of any one of embodiments 1 to 6, wherein all alkyl groups and alkylene groups present in the at least one amine of general formula (I) are C1-C6 groups, preferably C1-C4 groups.
8. The process of any one of embodiments 1 to 7, wherein if X is C, two, one or none of Y^{A1}, Y^{B1}, Y^{C1} and Y^{D1} are H, preferably wherein one or none of Y^{A1}, Y^{B1}, Y^{C1} and Y^{D1} is H, more preferably wherein none of Y^{A1}, Y^{B1}, Y^{C1} and Y^{D1} is H.
9. The process of any one of embodiments 1 to 7, wherein if X is Si or HC-CH, none of Y^{A1}, Y^{B1}, Y^{C1} and Y^{D1} is H.
10. The process of any one of embodiments 1 to 8 wherein X is C and a1 + b1 + c1 + d1 = 2.
11. The process of any one of embodiments 1 to 7 or 9, wherein X is Si and a1 +b1 +c1 + d1 ≥ 2.
12. The process of any one of embodiments 1 to 7 or 9, wherein X is HC-CH and a1 + b1 + c1 + d1 = 2 or a1 + b1 + c1 + d1 = 4.
13. The process of any one of embodiments 1 to 12, wherein the at least one amine of general formula (I) does not contain hydroxyl groups and/or aromatic groups.
14. The process of embodiment 13, wherein in the at least one amine of general formula (I) one or more amino groups are NH₂ groups that preferably are terminal NH₂ groups, and/or the at least one amine of general formula (I) contains 2 to 4 amino groups.
15. The process of any one of embodiments 1 to 14, wherein the at least one amine of general formula (I), the at least one intermediate (!1) and the at least one polyamine compound (II) do not contain halogen and sulfur atoms, do not contain aromatic groups and do contain at most two heterocyclic groups.
16. The process of embodiment 15, wherein the at least one amine of general formula (I), the at least one intermediate (!1) and the at least one polyamine compound (II) are formed solely of C, H, O, N, and optionally additionally one or two, preferably one, Si atoms.
17. The process of any one of embodiments 1 to 16, preferably of embodiment 15 or 16, wherein when X is C, then the at least one amine of general formula (I), the at least one intermediate (!1) and the at least one polyamine compound (II) have a molecular weight of at most 480 g/mol, preferably of at most 460 g/mol, more preferably of at most 440 g/mol.
18. The process of any one of embodiments 1 to 17, wherein step a) is carried out at a temperature in the range of from 5 to 200 °C, preferably at a temperature in the range of from 30 to 180 °C, more preferably at a temperature in the range of from 50 to 150 °C, and/or step a) is carried out at a pressure in the range of from 1 to 200 bar(abs), preferably at a pressure in the range of from 1 to 150 bar(abs), more preferably at a pressure in the range of from 1 to 100 bar(abs).
19. The process of any one of embodiments 1 to 18, wherein step a) is carried out in the presence of an alkoxylation catalyst, wherein the alkoxylation catalyst is preferably selected from the group consisting of water and heterogeneous catalysts, more preferably selected from the group consisting of water and ion exchange resins and zeolites, the zeolites optionally being doped with rare-earth elements, wherein more preferably, the catalyst is water.
20. The process of any one of embodiments 1 to 19, wherein step a) is carried out at a molar ratio of alkylene oxide relative to the amino groups of the at least one amine of general formula (I) of from 0.50 to 1.50, preferably of from 0.75 to 1.25, preferably of from 0.90 to 1.10.
21. The process of any one of embodiments 1 to 20, wherein step b) is carried out at a temperature in the range of from 150 to 260 °C, preferably in the range of from 170 to 240 °C, more preferably in the range of from 190 to 220 °C, and/or wherein step b) is carried out at a pressure in the range of from 1 to 325 bar(abs), preferably in the range of from 20 to 220 bar(abs), more preferably in the range of from 60 to 190 bar(abs).
22. The process of any one of embodiments 1 to 21, wherein in step b), the at least one first intermediate (I1) and the one or more of ammonia and at least one amine HNR'R" are employed at a molar ratio of the one or more of ammonia and at least one amine HNR'R" relative to the hydroxyl groups in the at least one first intermediate (I1) in the range of from 0.1 to 50.0, preferably in the range of from 0.5 to 40.0, more preferably in the range of from 1.0 to 30.0.
23. The process of any one of embodiments 1 to 22, wherein the catalyst used for the amination in step b) is a heterogeneous catalyst, preferably comprising copper, nickel and/or cobalt together with or without one or more of the noble metals rhodium, iridium, palladium, platinum, silver and/or gold, with or without one or more of iron, manganese, ruthenium, rhenium, molybdenum, tin, lanthanum, more preferably comprising copper, nickel and/or cobalt, more preferably comprising copper, nickel and cobalt, more preferably comprising either oxygen compounds of aluminum, of copper, of nickel and of cobalt, and, in the range from 0.2 to 5.0% by weight, of oxygen compounds of tin, calculated as SnO, previous to the activation of the catalyst by hydrogen, or oxygen compounds of aluminum, of copper, of nickel, of cobalt and of tin, and in the range from 0.2 to 5.0% by weight of oxygen compounds of yttrium, lanthanum, cerium and/or hafnium, calculated as Y₂O₃, La₂O₃, Ce₂O₃ or Hf₂O₃, respectively, previous to the activation of the catalyst by hydrogen.
24. An intermediate (I1), obtainable or obtained by the process according to any one of embodiments 1 to 23.
25. An intermediate (!1) as defined in any one of embodiments 1 to 23, preferably the intermediate according to embodiment 24, wherein at least one, preferably terminal, group NH of the amine of general formula (I) is converted to a group N-CH₂-CHR-OH or N-CHR-CH₂-OH with R being H or methyl or ethyl, preferably N-CH₂-CH₂-OH.
26. The intermediate (!1) of embodiment 25, wherein in said intermediate (!1) at least two, preferably terminal, groups NH of the amine of general formula (I) are converted to groups N-CH₂-CHR-OH or N-CHR-CH₂-OH with R being H or methyl or ethyl, preferably N-CH₂-CH₂-OH.
27. A polyamine compound (II), obtainable or obtained by the process according to any one of embodiments 1 to 23, that preferably demonstrates at least 20%, preferably at least 40% or more preferably at least 60% biodegradability according to the OECD 301 A, B, C, D, E or For OECD 302 B, preferably OECD 301 F, standard within 56 days, preferably within 28 days.
28. A polyamine compound (II) as defined in one of embodiments 1 to 23.
29. The polyamine compound (II) according to embodiment 27 or 28, comprising at least two, preferably terminal, groups NH or NR'.
30. Use of a polyamine compound (II) according to any one of embodiments 27 to 29 in or as co-reactant, hardener or curative for epoxy resins.
31. An epoxy resin composition, containing a polyamine compound (II) according to any one of embodiments 27 to 29, preferably as co-reactant, hardener or curative.
32. An epoxy resin composition, containing a polyamine compound (II) according to any one of embodiments 27 to 29, reacted as co-reactant, hardener or curative with an epoxy resin.
33. Use of a polyamine compound (II) according to any one of embodiments 27 to 29 or of compounds prepared therewith, for flotation; corrosion inhibition; asphalt emulsification; fertilizer-anticaking agents; additives for preventing caking of powdered minerals; emulsifiers, adjuvants, and intermediates in pesticide production; additives in plastic formulations; pigment-grinding aids; dispersants for pigments in paints, coatings, and magnetic tape; thickening agents in drilling muds, oil-based coatings and the paint industry; biocidal applications in households, swimming pools and oil fields; lubricants; petroleum additives; deicers for motor fuels; catalysis (such as in polyurethane formation); manufacturing of polyurethanes; intermediates for dyes, and of a polyamine compound (II) according to any one of embodiments 27 to 29 which contains tertiary amino groups as or for producing cleavable PU catalysts which are optionally incorporated due to additional functional groups or upon cleavage.
34. A process, preferably according to any one of embodiments 1 to 23, comprising the step of converting a chemical material obtainable by or obtained by the process according to any one of embodiments 1 to 23 to obtain a product Ω.
35. The process of embodiment 34, wherein the product Ω is selected from:
   - building block or monomer; or
   - polymer, preferably polymer A, polymer composition, preferably polymer composition A, or polymer product, preferably polymer product A; or
   - agrochemical composition, agrochemical formulation auxiliary or agrochemically active ingredient; or
   - active pharmaceutical ingredient or intermediate thereof, pharmaceutical excipient, animal feed additive, human food additive, dietary supplements, aroma chemical or aroma composition; or
   - aqueous polymer dispersion, preferably polyurethane or polyurethane - poly(meth)acrylate hybrid polymer dispersion, emulsion, binder for paper and fiber coatings, UV-curable acrylic polymer for hot melts and coatings polyisocyanates, hyperbranched polyester polyol, polymeric dispersant for inorganic binder compositions, unsaturated polyester polyol or 100% curable composition; or
   - cosmetic surfactant, emollient, wax, cosmetic polymer, UV filter, further cosmetic ingredient or composition or formulation thereof; or
   - polymer B, polymer composition B, coating composition, other functional composition, foil, molded body, coating or coated substrate.
36. The process of embodiment 34 or 35, wherein the content of the chemical material in the product Ω is 1 weight-% or more, preferably 2 weight-% or more, more preferably 5 weight-% or more, more preferably 15 weight-% or more, more preferably 30 weight-% or more, more preferably 40 weight-% or more, more preferably 60 weight-% or more, more preferably 80 weight-% or more, more preferably 90 weight-% or more, more preferably 95 weight-% or more; and/or
   wherein the content of the chemical material in the product Ω is 100 weight-% or less, preferably 95 weight-% or less, more preferably 90 weight-% or less, more preferably 50 weight-% or less, more preferably 25 weight-% or less, more preferably 10 weight-% or less; and preferably wherein the content is determined based on identity preservation and/or segregation and/or mass balance and/or book and claim chain of custody models, preferably based on mass balance, preferably the International Sustainability and Carbon Certification (ISCC) standard.

The invention is further illustrated by the following synthesis procedure.

### General synthesis procedure

The following section describes a general method for the synthesis of functionalized cleavable amines. The technologies used in this patent application describe the addition of alkylene oxide (ethylene oxide (EO), propylene oxide (PO) or butylene oxide (BO)) to a di- or higher polyamine, followed by alcohol amination of the resulting hydroxyl groups to yield the corresponding ethylamine-extended (with potentially further alkyl substituents in the C2-chain based on the respective alkylene oxide used) di- or higher polyamine (functionalized cleavable amine).

The functionalized cleavable amine is to be synthesized in a two-step process. Starting from the cleavable amine and alkylene oxide (AO), an addition reaction is used to synthesize a multi-alkoxylated product as the desired intermediate for alcohol amination. This intermediate is then subjected to catalytic alcohol amination to yield the functionalized cleavable amine.

In the first reaction stage, AO is added to a cleavable amine. The product spectrum significantly depends on the molar ratio of N-H functionality and AO. Since amines have a significantly higher nucleophilicity than alcohols, the corresponding overalkoxylated products are only detected in small amounts. In the case of higher alkylene oxides (PO, BO), regioisomers occur, where, due to steric effects, the addition of the amine is preferred to the sterically less hindered C atom of the oxirane ring. Consequently, the 1-amino-2-hydroxy-propanol or -butanol derivative is preferably formed. Due to the complexity of the product mixture, the obtained crude product can be optionally purified, e.g. via distillation. The reaction can be optionally catalyzed. For this purpose, for instance water, which is initially added to the amine, or fixed-bed catalysts such as organic ion-exchange resins, acidic inorganic clays or zeolites activated by rare-earth metals can be used as catalysts.

The addition of alkylene oxide is carried out either continuously, in semi-batch mode or in batch mode. Initially, the semi-batch synthesis was pursued, and only this variation will be described below.

In the semi-batch synthesis, the cleavable amine is introduced into an addition reactor and optionally mixed with water under stirring. The reaction takes place (mainly) in the liquid phase. The cleavable amine or mixture is heated to the reaction temperature, and then AO is added while stirring. The dosing rate should be chosen in a way that prevents AO accumulation (also in gas phase) and allows the desired reaction temperature to be maintained. Therefore, the dosing rate depends on the reaction kinetics and the cooling capacity of the reactor. Ensuring the initiation of the reaction (e.g., through a minimum temperature that has to be achieved) is crucial. The reaction was carried out at a temperature in the range of from 50 to 150 °C and a pressure in the range of from 1 to 100 bar(abs). After the AO dosing is completed, the reaction mixture is further stirred at the reaction temperature for a defined period to ensure complete conversion of AO (preferred residual AO concentration: less than 0.1 GC area%).

In the second reaction stage, the reaction mixture is converted into the functionalized cleavable amine raw product through catalytic alcohol amination in the presence of a catalyst. Since the reaction proceeds via borrowing hydrogen or hydrogen autotransfer mechanism, formally no hydrogen is needed, but hydrogen is added optionally to the reaction mixture.

The reaction may be carried out using stirred tank reactors, fixed-bed tube reactors and multitube reactors. It may be carried out in batch, semi-batch and continuous mode and with or without recycling of the crude reaction mixture. In a preferred embodiment, the reaction is carried out in continuous mode in a fixed-bed tube reactor.

The alcohol amination is performed using a fixed-bed catalyst. Preferably, the reaction is carried out in the presence of a hydrogenation/dehydrogenation catalyst, for example in the presence of a copper-containing hydrogenation/dehydrogenation catalyst. The catalyst may be applied to a support, for example an alumina support. In one embodiment, a supported copper-, nickel- and cobalt-containing hydrogenation/dehydrogenation catalyst is used, wherein the catalytically active material of the catalyst, before the reduction thereof with hydrogen, comprises oxygen compounds of aluminum, of copper, of nickel and of cobalt, and in the range from 0.2 to 5.0% by weight of oxygen compounds of tin, calculated as SnO. In a preferred embodiment, a catalyst according to the catalysts claimed in WO 2011/067199 is used, particularly a catalyst according to WO 2011/067199, example 5. Optionally, the catalyst is activated prior to the reaction by using hydrogen gas and temperatures above 150°C, preferably above 200°C, more preferably above 220°C.

The reaction is carried out by feeding the reactant, along with ammonia or primary amine or secondary amine (preferably ammonia or monomethylamine or dimethylamine), and optionally hydrogen and solvent, into the reactor. In a preferred embodiment, the reaction is carried out at a temperature of 150 to 260 °C. In an especially preferred embodiment, the reaction is carried out at a temperature of 170 to 240 °C. In a most preferred embodiment, the reaction is carried out at a temperature of 190 to 220 °C. The reaction may be carried out at a pressure in the range of from 1-325 bar(abs), in liquid and vapour phase. In a preferred embodiment, the reaction is carried out at a pressure in the range of from 20 to 220 bar (abs). In an especially preferred embodiment, the reaction is carried out at a pressure in the range of from 60 to 190 bar (abs).

The load of the catalyst by starting material (reaction product obtained by addition of alkylene oxide to cleavable amine) may be varied from 0.01-2 kg/Uh, in a preferred embodiment it is in the range of 0.1-1.0 kg/Uh, and in an especially preferred embodiment it ranges from 0.2-0.8 kg/L/h.

Optionally, the crude product is filtered to withhold catalyst particles and fines. Optionally, in case ammonia was used for the alcohol amination, excess ammonia is distilled and recycled back to the reaction. Optionally, the crude product is purified, for instance, by distillation.

The distillation of the raw product can be carried out continuously or in a batch mode. Initially, the batch distillation was pursued, and only this variation will be described below.

The collected and homogenized raw product is introduced into the bottom of the distillation column. The distillation is conducted under reduced pressure, with a minimal pressure, preferably less than 20 mbar sump pressure, being preferred to reduce thermal stress on the distillation sump and to provide a greater margin to the onset of decomposition reactions. By increasing the temperature at isobaric conditions, the light-boiling components are gradually removed.

Subsequently, the product is distilled and collected. After the desired amount of valuable product is obtained, the vapor supply is closed, and the vacuum is broken. Depending on the safety concept of the distillation, either multiple batches of raw material can be filled into the column and distilled before the sump is removed, or the sump has to be removed after each distillation. The light- and heavy-boiling components are sent for incineration. The obtained purified product is stored until further use. The concentration of the obtained functionalized cleavable amine in the purified product is preferably greater than 98.5 GC area%.

The products of the alcohol amination reaction, functionalized cleavable amines with acetal, ketal, or silyl ketal functionality in the backbone, are partially thermolabile. If these raw products cannot be distilled without significant decomposition, they can be used in further applications without further workup. This is also possible if high purity is not required for further use or if the side components do not impair or even enhance the final product performance. For example, a raw product composition containing at least 50 wt.%, preferably at least 60 wt.%, more preferably at least 70 wt.% compound (II) can be employed.

## Claims

1. A process for preparing polyamines via alkylene oxide addition to an amine followed by alcohol amination, comprising
a) reacting at least one amine of general formula (I) 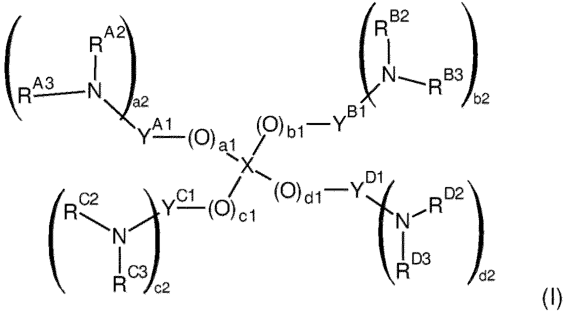 with at least one C2-C4-alkylene oxide, thereby obtaining at least one first intermediate (I1), wherein the amine of general formula (I) comprises at least one, preferably terminal, group NH which is reacted with the C2-C4-alkylene oxide, thereby obtaining at least one first intermediate (!1), wherein at least one, preferably terminal, group NH of the amine of general formula (I) is converted to at least one group N-CH₂-CHR-OH or N-CHR-CH₂-OH with R being H or methyl or ethyl, preferably N-CH₂-CH₂-OH;
b) aminating the at least one first intermediate (I1) with at least one of ammonia, primary and secondary amines HNR'R" with R' being H, methyl, ethyl, propyl or isopropyl and R" being H, methyl, ethyl, propyl or isopropyl, or mixtures thereof, to form at least one polyamine compound (II), comprising converting the at least one group N-CH₂-CHR-OH or N-CHR-CH₂-OH, preferably the at least one group N-CH₂-CH₂-OH to the at least one group N-CH₂-CHR-NR'R" or N-CHR-CH₂-NR'R", preferably to the at least one group N-CH₂-CH₂-NH₂; and
if at least one group N(-CH₂-CHR-OH)₂, N(-CHR-CH₂-OH)₂, or N(-CHR-CH₂-OH) (-CH₂-CHR-OH), preferably at least one group N(-CH₂-CH₂-OH)₂, is present in the at least one first intermediate (I1) and at least one of ammonia or primary amines H₂NR' with R' being H, methyl, ethyl, propyl or isopropyl, or mixtures thereof, are employed in step b),
then step b) further comprises the step of converting the at least one group N(-CH₂-CHR-OH)₂, N(-CHR-CH₂-OH)₂, or N(-CHR-CH₂-OH)(-CH₂-CHR-OH), preferably at least one group N(-CH₂-CH₂-OH)₂, in the at least one first intermediate (!1) with ammonia or primary amine H₂NR' with R' being H, methyl, ethyl, propyl or isopropyl, or mixtures thereof, to form at least one, optionally substituted, piperazine group -N(-CH₂-CHR-)₂NR', -N(-CHR-CH₂-)₂NR' or -N(-CH₂-CHR-)(-CHR-CH₂-)NR', preferably to form at least one piperazine group -N(-CH₂-CH₂-)₂NH as shown below; 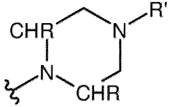 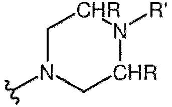 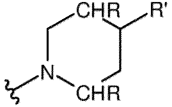 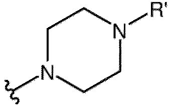
wherein X is C or Si or HC-CH;
wherein a1, a2, b1, b2, c1, c2, d1, d2 are independently of each other 0 or 1, with a1 + b1 + c1 + d1 ≥ 2 and a2 + b2 + c2 + d2 ≥ 2;
wherein, if a1 = a2 = 0, Y^{A1} is H or optionally substituted C1-C12 alkyl;
wherein, if a1 = 1 and a2 = 0, Y^{A1} is optionally substituted C1-C12 alkyl;
wherein, if a1 = a2 = 1, or if a1 = 0 and a2 = 1, Y^{A1} is optionally substituted C1-C12 alkylene;
wherein if b1 = b2 = 0, Y^{B1} is H or optionally substituted C1-C12 alkyl;
wherein if b1 = 1 and b2 = 0, Y^{B1} is optionally substituted C1-C12 alkyl;
wherein if b1 = b2 = 1, or if b1 = 0 and b2 = 1, Y^{B1} is optionally substituted C1-C12 alkylene;
wherein if c1 = c2 = 0, Y^{C1} is H or optionally substituted C1-C12 alkyl;
wherein if c1 = 1 and c2 = 0, Y^{C1} is optionally substituted C1-C12 alkyl;
wherein if c1 = c2 = 1, or if c1 = 0 and c2 = 1, Y^{C1} is optionally substituted C1-C12 alkylene;
wherein if d1 = d2 = 0, Y^{D1} is H or optionally substituted C1-C12 alkyl;
wherein if d1 = 1 and d2 = 0, Y^{D1} is optionally substituted C1-C12 alkyl;
wherein if d1 = d2 = 1, or if d1 = 0 and d2 = 1, Y^{D1} is optionally substituted C1-C12 alkylene;
wherein R^{A2}, R^{A3}, R^{B2}, R^{B3}, R^{C2}, R^{C3}, R^{D2}, R^{D3} are, independently of each other, H or optionally substituted C1-C12 alkyl, with at least one of R^{A2}, R^{A3}, R^{B2}, R^{B3}, R^{C2}, R^{C3}, R^{D2}, and R^{D3} being H;
wherein, if X is C or Si,
- Y^{A1} and Y^{B1} can be covalently linked to form a 5- or 6-membered ring with X, preferably when a1 = b1 = 1; and/or
- Y^{C1} and Y^{D1} can be covalently linked to form a 5- or 6-membered ring with X, preferably when c1 = d1 = 1;
wherein, if X is HC-CH and (O)ₐ₁(O)_{b1}X(O)_{c1}(O)_{d1} is 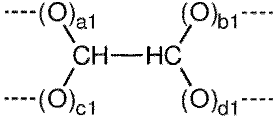
- Y^{A1} and Y^{C1} can be covalently linked to form a 5- or 6-membered ring with X, preferably when a1 = c1 = 1; and/or
- Y^{B1} and Y^{D1} can be covalently linked to form a 5- or 6-membered ring with X, preferably when b1 = d1 = 1; or
wherein, if X is HC-CH and (O)ₐ₁(O)_{b1}X(O)_{c1}(O)_{d1} is 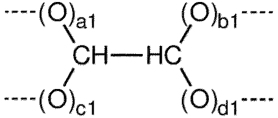
- Y^{A1} and Y^{B1} can be covalently linked to form a 5- or 6-membered ring with X, preferably when a1 = b1 = 1; and/or
- Y^{C1} and Y^{D1} can be covalently linked to form a 5- or 6-membered ring with X, preferably when c1 = d1 = 1,
wherein preferably the amine of general formula (I) comprises at least two, preferably terminal, groups NH which are reacted with the alkylene oxide.

2. The process of claim 1, having one or more of the following features:
- if a1 = 1, then a2 = 1; if b1 = 1, then b2 = 1; if c1 = 1, then c2 = 1; and if d1 = 1, then d2 = 1;
- R^{A2}, R^{B2}, R^{C2} and R^{D2} are H, methyl, ethyl, propyl or isopropyl, preferably H, and R^{A3}, R^{B3}, R^{C3}, R^{D3} are H; or
R^{A2}, R^{B2}, R^{C2} and R^{D2} are H, methyl, ethyl, propyl or isopropyl, preferably H, and R^{A3}, R^{B3}, R^{C3}, R^{D3} are methyl; or
R^{A2}, R^{B2}, R^{C2} and R^{D2} are H, methyl, ethyl, propyl or isopropyl, preferably H, and R^{A3}, R^{B3}, R^{C3}, R^{D3} are ethyl; or
R^{A2}, R^{B2}, R^{C2} and R^{D2} are H, methyl, ethyl, propyl or isopropyl, preferably H, and R^{A3}, R^{B3}, R^{C3}, R^{D3} are propyl; or
R^{A2}, R^{B2}, R^{C2} and R^{D2} are H, methyl, ethyl, propyl or isopropyl, preferably H, and R^{A3}, R^{B3}, R^{C3}, R^{D3} are isopropyl;
- all alkyl groups and alkylene groups present in the at least one amine of general formula (I) are non-branched groups;
- all alkyl groups and alkylene groups present in the at least one amine of general formula (I) are C1-C6 groups, preferably C1-C4 groups;
- if X is C, two, one or none of Y^{A1}, Y^{B1}, Y^{C1} and Y^{D1} are H, preferably wherein one or none of Y^{A1}, Y^{B1}, Y^{C1} and Y^{D1} is H, more preferably wherein none of Y^{A1}, Y^{B1}, Y^{C1} and Y^{D1} is H;
- if X is Si or HC-CH, none of Y^{A1}, Y^{B1}, Y^{C1} and Y^{D1} is H;
- X is C and a1 + b1 + c1 + d1 = 2;
- X is Si and a1 + b1 + c1 + d1 ≥ 2;
- X is HC-CH and a1 + b1 + c1 + d1 = 2 or a1 + b1 + c1 + d1 = 4.

3. The process of claim 1 or 2, having one or more of the following features:
- the at least one amine of general formula (I) does not contain hydroxyl groups and/or aromatic groups,
- the at least one amine of general formula (I), the at least one intermediate (I1) and the at least one polyamine compound (II) are formed solely of C, H, O, N, and optionally additionally one or two, preferably one, Si atoms,
- in the at least one amine of general formula (I) one or more amino groups are NH₂ groups that preferably are terminal NH₂ groups, and/or the at least one amine of general formula (I) contains 2 to 4 amino groups,
- the at least one amine of general formula (I), the at least one intermediate (!1) and the at least one polyamine compound (II) do not contain halogen and sulfur atoms, do not contain aromatic groups and do contain at most two heterocyclic groups.

4. The process of any one of claims 1 to 3, wherein when X is C, the at least one amine of general formula (I), the at least one intermediate (!1) and the at least one polyamine compound (II) have a molecular weight of at most 480 g/mol, preferably of at most 460 g/mol, more preferably of at most 440 g/mol.

5. The process of any one of claims 1 to 4, comprising one or more of the following features:
- step a) is carried out at a temperature in the range of from 5 to 200 °C, preferably at a temperature in the range of from 30 to 180 °C, more preferably at a temperature in the range of from 50 to 150 °C;
- step a) is carried out at a pressure in the range of from 1 to 200 bar(abs), preferably at a pressure in the range of from 1 to 150 bar(abs), more preferably at a pressure in the range of from 1 to 100 bar(abs);
- step a) is carried out in the presence of an alkoxylation catalyst, wherein the alkoxylation catalyst is preferably chosen from the group consisting of water and heterogeneous catalysts, more preferably chosen from the group consisting of water and ion exchange resins and zeolites, the zeolites optionally being doped with rare-earth elements, most preferably water being chosen as catalyst;
- step a) is carried out at a molar ratio of alkylene oxide relative to the amino groups of the at least one amine of general formula (I) of from 0.50 to 1.50, preferably of from 0.75 to 1.25, preferably of from 0.90 to 1.10;
- step b) is carried out at a temperature in the range of from 150 to 260 °C, preferably in the range of from 170 to 240 °C, more preferably in the range of from 190 to 220 °C;
- step b) is carried out at a pressure in the range of from 1 to 325 bar(abs), preferably in the range of from 20 to 220 bar(abs), more preferably in the range of from 60 to 190 bar(abs);
- in step b), the at least one first intermediate (I1) and the one or more of ammonia and at least one amine HNR'R" are employed at a molar ratio of the one or more of ammonia and at least one amine HNR'R" relative to the hydroxyl groups in the at least one first intermediate (I1) in the range of from 0.1 to 50.0, preferably in the range of from 0.5 to 40.0, more preferably in the range of from 1.0 to 30.0.

6. The process of any one of claims 1 to 5, wherein the catalyst used for the amination in step b) is a heterogeneous catalyst, preferably comprising copper, nickel and/or cobalt together with or without one or more of the noble metals rhodium, iridium, palladium, platinum, silver and/or gold, with or without one or more of iron, manganese, ruthenium, rhenium, molybdenum, tin, lanthanum, more preferably comprising copper, nickel and/or cobalt, more preferably comprising copper, nickel and cobalt, more preferably comprising either oxygen compounds of aluminum, of copper, of nickel and of cobalt, and, in the range from 0.2 to 5.0% by weight, of oxygen compounds of tin, calculated as SnO, previous to the activation of the catalyst by hydrogen, or oxygen compounds of aluminum, of copper, of nickel, of cobalt and of tin, and in the range from 0.2 to 5.0% by weight of oxygen compounds of yttrium, lanthanum, cerium and/or hafnium, calculated as Y₂O₃, La₂O₃, Ce₂O₃ or Hf₂O₃, respectively, previous to the activation of the catalyst by hydrogen.

7. An intermediate (I1), obtainable or obtained by the process according to any one of claims 1 to 6.

8. An intermediate (I1), preferably obtainable or obtained by a process according to any one of claims 1 to 6, wherein at least one, preferably terminal, group NH of the amine of general formula (I) is converted to a group N-CH₂-CHR-OH or N-CHR-CH₂-OH with R being H or methyl or ethyl, preferably N-CH₂-CH₂-OH, wherein in said intermediate (11), preferably at least two, preferably terminal, groups NH of the amine of general formula (I) are converted to groups N-CH₂-CHR-OH or N-CHR-CH₂-OH with R being H or methyl or ethyl, preferably N-CH₂-CH₂-OH.

9. A polyamine compound (II), obtainable or obtained by the process according to any one of claims 1 to 6.

10. A polyamine compound (II) as defined in one of claims 1 to 6, preferably the polyamine compound of claim 9, comprising at least two, preferably terminal, groups NH or NR'.

11. Use of a polyamine compound (II) according to claim 9 or 10 in or as co-reactant, hardener or curative for epoxy resins.

12. An epoxy resin composition, containing a polyamine compound (II) according to claim 9 or 10, preferably as co-reactant, hardener or curative.

13. An epoxy resin composition, containing a polyamine compound (II) according to claim 9 or 10, reacted as co-reactant, hardener or curative with an epoxy resin.

14. Use of a polyamine compound (II) according to claim 9 or 10 or of compounds prepared therewith, for flotation; corrosion inhibition; asphalt emulsification; fertilizer-anticaking agents; additives for preventing caking of powdered minerals; emulsifiers, adjuvants, and intermediates in pesticide production; additives in plastic formulations; pigment-grinding aids; dispersants for pigments in paints, coatings, and magnetic tape; thickening agents in drilling muds, oil-based coatings and the paint industry; biocidal applications in households, swimming pools and oil fields; lubricants; petroleum additives; deicers for motor fuels; catalysis (such as in polyurethane formation); manufacturing of polyurethanes; intermediates for dyes, and of a polyamine compound (II) according to claim 9 or 10 which contains tertiary amino groups as or for producing cleavable PU catalysts which are optionally incorporated due to additional functional groups or upon cleavage.

15. A process, preferably according to any one of claims 1 to 6, comprising the step of converting a chemical material obtainable by or obtained by the process according to any one of claims 1 to 6 to obtain a product Ω.
